# EUROPÄISCHE PATENTANMELDUNG

(11) **EP 0 142 684 A2**
(43) Veröffentlichungstag der Anmeldung: **29.05.1985**
(21) Anmeldenummer: 84111704.7
(22) Anmeldetag: 01.10.1984
(51) Int. Cl.: C07D 405/06, C07D 307/28, C07C 49/16, A01N 43/50, A01N 43/653

(54) **Azolyl-tetrahydrofuran-2-yliden-methan-Derivate**

(30) Priorität: 11.10.1983 DE 3336861
(71) Anmelder: BAYER AG, 51368 Leverkusen (DE)
(72) Erfinder: Elbe, Hans-Ludwig, Dr., D-5600 Wuppertal 11 (DE); Jautelat, Manfred, Dr., D-5093 Burscheid (DE); Büchel, Karl Heinz, Prof. Dr., D-5093 Burscheid (DE); Brandes, Wilhelm, Dr., D-5653 Leichlingen 1 (DE); Hänssler, Gerd, Dr., D-5090 Leverkusen 1 (DE); Reinecke, Paul, Dr., D-5090 Leverkusen 3 (DE)

(57) **Zusammenfassung**

Azolyl-tetrahydrofuran-2-yliden-methan- Derivate der allgemeinen Formel (I) 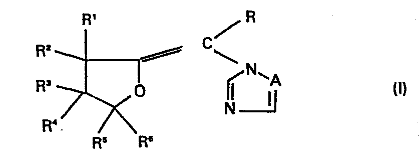 in welcher
A für ein Stickstoffatom oder die CH-Gruppe steht,
R fürAlkyl,Alkenyl,Alkinyl,Alkoxyalkyl,Alkylthio-alkyl,; Halogenalkyl, Halogenalkoxyalkyl, Halogenalkyl-thioalkyl, Halogenalkenyl, gegebenenfalls substituiertes Aralkyl, gegebenenfalls substituiertes Aryloxyalkyl, gegebenenfalls substituiertes Arylthioalkyl, gegebenenfalls substituiertes Cycloalkyl öder gegebenenfalls substituiertes Cycloalkylalkyl' steht,
R' bis R" gleich oder verschieden sind und für Wasserstoff, Alkyl, Halogenalkyl oder Halogen stehen, wobei maximal bis zu 3 Substituenten für Halogen bzw. Halogenalkyl stehen, oder
R¹ und R² gemeinsam mit dem Kohlenstoffatom, an das sie gebunden sind, für gegebenenfalls substituiertes Cycloalkyl stehen oder
R² und R³ gemeinsam mit den Kohlenstoffatomen, an die sie gebunden sind, für gegebenenfalls substituiertes Cycloalkyl stehen,

und deren Säureaddditions-Salze und Metallsalz-komplexe sowie ihre Verwendung als Fungizide.

Die neuen Azolyl-tetrahydrofuran-2-yliden- methan-Derivate können können aus geeigneten Azolyl-(2-hydroxytetrahydrofuran-2-yl)- methan-Derivaten durch Wasserabspaltung erhalten werden.

## Beschreibung

Die vorliegende Erfindungs betrifft neue substituierte Azolyltetrahydrofuran-2-yliden-methan-Derivate, ein Verfahren zu ihrer Herstellung sowie ihre Verwendung als Fungizide.

Es ist bereits bekannt geworden, daß bestimmte Azolylalkenole, wie z.B. im Phenoxyteil substituierte 1-(Imidazol-1-yl)-bzw. 1-(1,2,4-Triazot-1-yt)-2-phenoxy-4,4-dimethyL-1-penten-3-ole, gute fungizide Eigenschaften aufweisen(vergleiche DE-OS 29 28 967). Außerdem ist bereits bekannt, daß Disulfide, wie z.B. Zinkethylen-1,2-bisdithio- carbamidat, gute Mittel zur Bekämpfung von pilzlichen Pflanzenkrankheiten sind (vergleiche R.Wegler,"Chemie der Pflanzenschutz- und Schädlingsbekämpfungsmittel; Band 2, S. 59ff, Springer-Verlag 1970). Die Wirkung dieser Verbindungen ist jedoch in bestimmten Indikationsbereichen, insbesondere bei niedrigen Aufwandmengen und -konzentrationen, nicht immer voll befriedigend.

Es wurden neue substituierte Azolyl-tetrahydrofuran-2-ytiden- methan-Derivate der allgemeinen Formel (I) 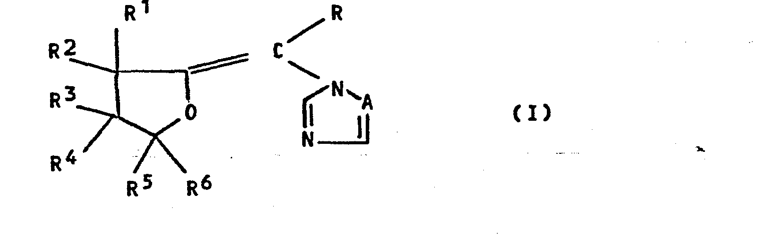 in welcher
A für ein Stickstoffatom oder die CH-Gruppe steht,
R für Alkyl, Alkenyl, Alkinyl, Alkoxyalkyl, Alkylthioalkyl, Hatogenalkyl, Halogenalkoxyalkyl, Halogenalkyl- thioalkyl, Halogenalkenyl, gegebenenfalls substituiertes Aralkyl, gegebenenfalls substituiertes Aryloxyalkyl, gegebenenfalls substituiertes Arylthioalkyl, gegebenenfalls substituiertes Cycloalkyl oder gegebenenfalls sub₋ stituiertes Cycloalkylalkyl steht,
R¹ bis R⁶ gleich oder verschieden sind und für Wasserstoff, Alkyl, Halogenalkyl oder Halogen stehen, wobei maximal bis zu 3 Substituenten für HaLogen bzw.Halogenalkyl stehen, oder
R¹ und R² gemeinsam mit dem Kohlenstoffatom, an das sie gebunden sind,für gegebenenfalls substituiertes Cycloalkyl stehen, oder
R² und R³ gemeinsam mit den Kohlenstöffatomen, an die sie gebunden sind, für gegebenenfalls substituiertes Cycloalkyl stehen,

und deren Säureadditions-Salze und Metallsalz-komplexe gefunden.

Die Verbindungen der Formel (I) können in zwei geometrischen Isomerenformen vorliegen, je nach Anordnung der Gruppen, die an die Doppelbindung gebunden sind; vorzugsweise fallen sie in einem wechselnden Isomerenverhättnis an. Die vorliegende Erfindung betrifft sowohl die einzelnen Isomeren als auch die Isomerengemische.

Weiterhin wurde gefunden, daß man die substituierten Azolyl-tetrahydrofuran-2-yliden-methan-Derivate der Formel (I) erhält, wenn man Azolyl-(2-hydroxytetrahydro- fiuran-2-yl)-methan-Derivate der Formel (II) 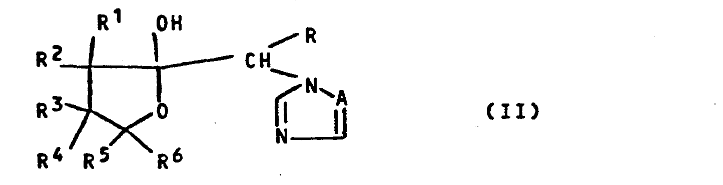 in welcher
A,R und R¹ bis R⁶ die oben angegebene Bedeutung haben,

in Gegenwart eines Verdünnungsmittels und in Gegenwart eines sauren Katalysators zur Wasserabscheidung erhitzt.

An die so erhaltenen Verbindungen der Formel (I) kann gegebenenfalls anschließend eine Säure oder ein Metallsalz addiert werden.

Außerdem wurde gefunden, daß die neuen substituierten Azolyl-tetrahydrofuran-2-yliden-methan-Derivate der Formel (I) starke fungizide Eigenschaften aufweisen. Ueberraschenderweise zeigen die erfindungsgemäßen Verbindungen der Formel (I) bessere fungizide Wirkungen als die aus dem Stand der Techik bekannten, im Phenoxyteil substituierten, 1-(Imidazol-1-yl)-bzw.1-(1,2,4-Triazol-1-yl)-2-phenoxy-4,4-dimethyl-1-penten-3-ole und als das ebenfalls bekannte Zinkethylen-1,2-bisdithiocarbamidat. Die erfindungsgemäßen Wirkstoffe stellen somit eine Bereicherung der Technik dar.

Die erfindungsgemäßen substituierten Azolyl-tetrahydrofuran-2-ytiden-methan-Derivate sind durch die Formet (1) allgemein definiert. In dieser Formel stehen vorzugsweise
A für ein Stickstoffatom oder die CH-Gruppe;
R für geradkettiges oder verzweigtes Alkyl mit 1 bis 12 Kohlenstoffatomen, für geradkettiges oder verzweigtes Alkenyl und Alkinyl mit jeweils 2 bis 12 Kohlenstoffatomen, für geradkettiges oder verzweigtes Alkoxyalkyl und Alkylthioalkyl mit jeweils 1 bis 6 Kohlenstoffatomen in jedem Alkylteil, für Halogenalkyl, Halogenalkoxyalkyl und Halogenalklthioalkyl mit jeweils 1 bis 6 Kohlenstoffatomen in jedem Alkylteil und mit jeweils 1 bis 6 gleichen oder verschiedenen Halogenatomen, wie Fluor- und Chloratomen, für Halogenalkenyl mit 2 bis 6 KohLenstoffatomen und 1 bis 5 gleichen oder verschiedenen Halogenatomen, wie FLuor- und Chloratomen, ferner für jeweils gegebenenfalls einfach oder mehrfach, gleich oder verschieden im PhenyLteiL substituiertes Phenylalkyl, Phenoxyalkyl oder Phenylthioalkyl mit jeweils 1 bis 4 Kohlenstoffatomen im Alkylteil, wobei als Substituenten genannt seien:Halogen; Alkyl, Alkoxy und Alkylthio mit jeweils 1 bis 4 Kohtenstoffatomen; Halogenalkyl, Halogenalkoxy und Halogenalkylthio mit jeweils 1 bis 4 Kohlenstoffatomen und 1 bis 5 gleichen oder verschiedenen Halogenatomen; wie Fluor- und Chloratomen; Nitro; Cyano; sowie gegebenenfalls durch Halogen substituiertes Phenyt und Phenoxy; sowie für jeweils gegebenenfalls einfach oder mehrfach durch Alkyl mit 1 bis 4 Kohlenstoffatomen substituiertes Cycloalkyl und Cycloalkylalkyl mit jeweils 3 bis 7 Kohlenstoffatomen im Cycloalkylteil und 1 bis 4 Kohlenstoffatomen im Alkylteil;
R¹ bis R⁶, die gleich oder verschieden sein können, für Wasserstoff, geradkettiges oder verzweigtes Alkyl mit 1 bis 4 Kohlenstoffatomen oder für Halogenalkyl mit 1 oder 2 Kohlenstoffatomen und 1 bis 5 gleichen oder verschiedenen Halogenatomen, wie Fluor- und Chloratomen, wobei maximal bis zu 3 Substituenten für Halogenalkyl stehen,
R³ bis R⁶, die gleich oder verschieden sein können, außerdem für Halogen, wobei maximal bis zu 3 Substituenten diese Bedeutung haben; oder
R¹ und R² gemeinsam mit dem Kohlenstoffatom, an das sie gebunden sind, für gegebenenfalls durch Alkyl mit 1 oder 2 Kohlenstoffatomen substituiertes CycLoaLkyL mit 4 bis 7 Kohlenstoffatomen; oder
R² und R³ gemeinsam mit den Kohlenstoffatamen, an die sie gebunden sind, für gegebenenfalls durch Alkyl mit 1 oder 2 Kohlenstoffatomen substituiertes Cycloalkyl mit 4 bis 7 Kohlenstoffatomen.

Besonders bevorzugt sind diejenigen Verbindungen der Formel (I), in denen
A für ein Stickstoffatom oder die CH-Gruppe steht;
R für geradkettiges oder verzweigtes Alkyl mit 1 bis 8 Kohlenstoffatomen, für geradkettiges oder verzweigtes Alkenyl und Alkinyl mit jeweils 2 bis 6 Kohlenstoffatomen, für geradkettiges oder verzweigtes Alkoxyalkyl und Alkylthioalkyl mit jeweils 1 bis 4 Kohlenstoffatomen in jedem Alkylteil, für Halogenalkyl, Halogenalkoxyalkyl und Halogenalkylthioalkyl mit jeweils 1 bis 4 KohLenstoffatomen in jedem Alkylteil und mit jeweils 1 bis 5 Fluor- und/oder Chloratomen, für Halogenalkenyl mit 2 bis 4 Kohlenstoffatomen und 1 bis 3 Fluor- und/oder Chloratomen; ferner für jeweils gegebenenfalls einfach bis dreifach, aleich oder verschieden im Phenylteil substituiertes Phenylalkyl, Phenoxyalkyl oder Phenylthioalkyl mit jeweils 1 oder 2 Kohlenstoffiatomer im Alkylteil steht, wobei als Substituenten genannt seien: Fluor, Chtor, Methyl, Ethyl, Isopropyl, tert.-Butyl, Methoxy, Methylthio, Trifluormethyl, Trifluormethoxy, Trifluorme-th,ylthio, Nitro, Cyano, sowize gegebenenfalls durch Chlor substituiertes Phenyt und Phenoxy; sowie auch für jeweils gegebenenfalls durch Methyl und /oder Ethyl substituiertes Cyclohexyl oder Cyclohexylmethyl steht;
_{R}¹ bis R⁶, die gleich oder verschieden sein können, für Wasserstoff, Methyl, Ethyl oder Halogenmethyl mit 1 bis 3 gleichen oder verschiedenen Halogenatomen, wie Fluor-und Chloratomen stehen, wobei maximal drei Substituenten für Halogenmethyl stehen;
R³ bis R⁶, die gleich oder verschieden sein können, außerdem für Fluor, Chlor oder Brom stehen, wobei maximal bis zu drei Substituenten diese Bedeutung haben; oder
R¹ und R² gemeinsam mit dem Kohlenstoffatom, an das sie gebunden sind, für Cyclopentyl oder Cyclohexyl stehen; oder
R² und R³ gemeinsam mit den Kohlenstoffatomen, an die sie gebunden sind, für Cyclopentyl oder Cyclohexyl stehen. Im einzelnen seien außer den bei den Herstellungsbeispielen genannten Verbindungen die folgenden Verbindungen der allgemeinen formel (I) genannt (wobei A sowohl für ein Stickstoffatom als auch die CH-Gruppe steht): 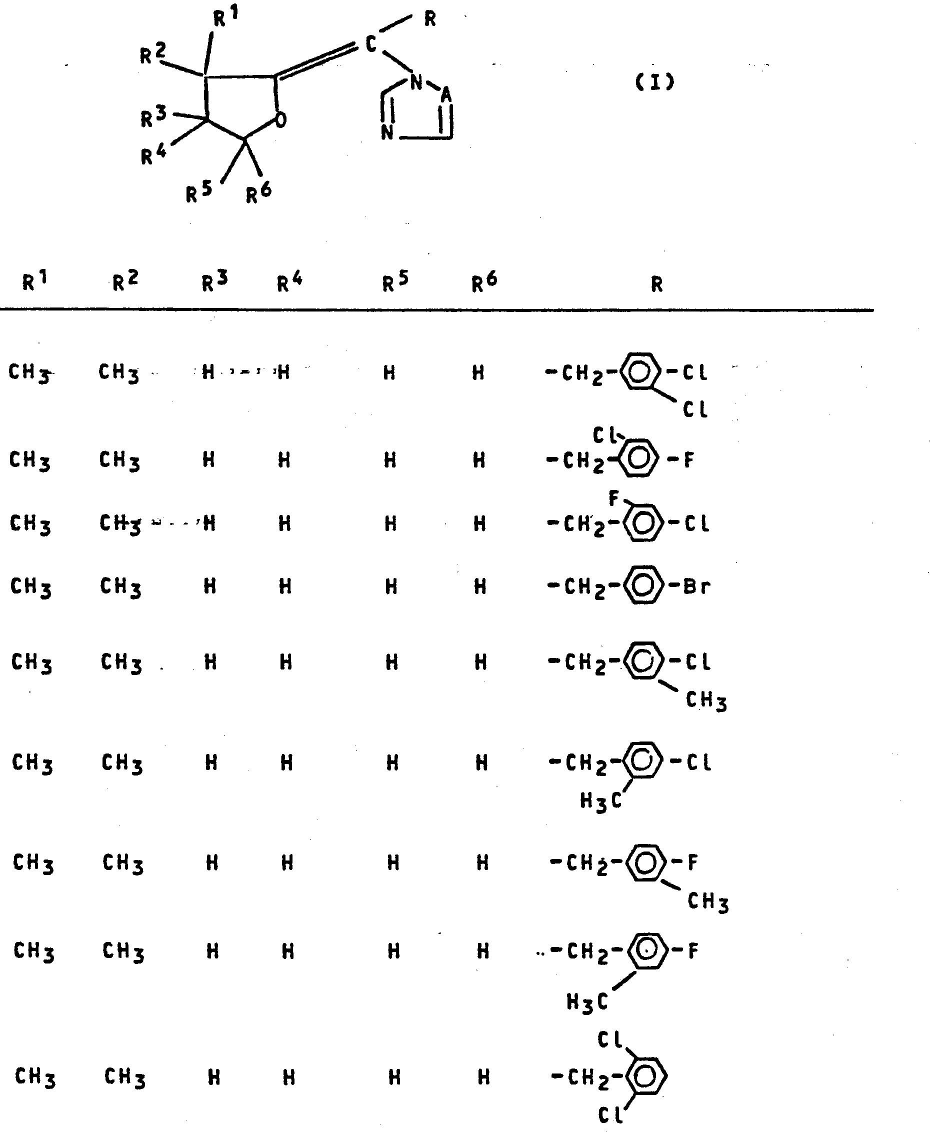 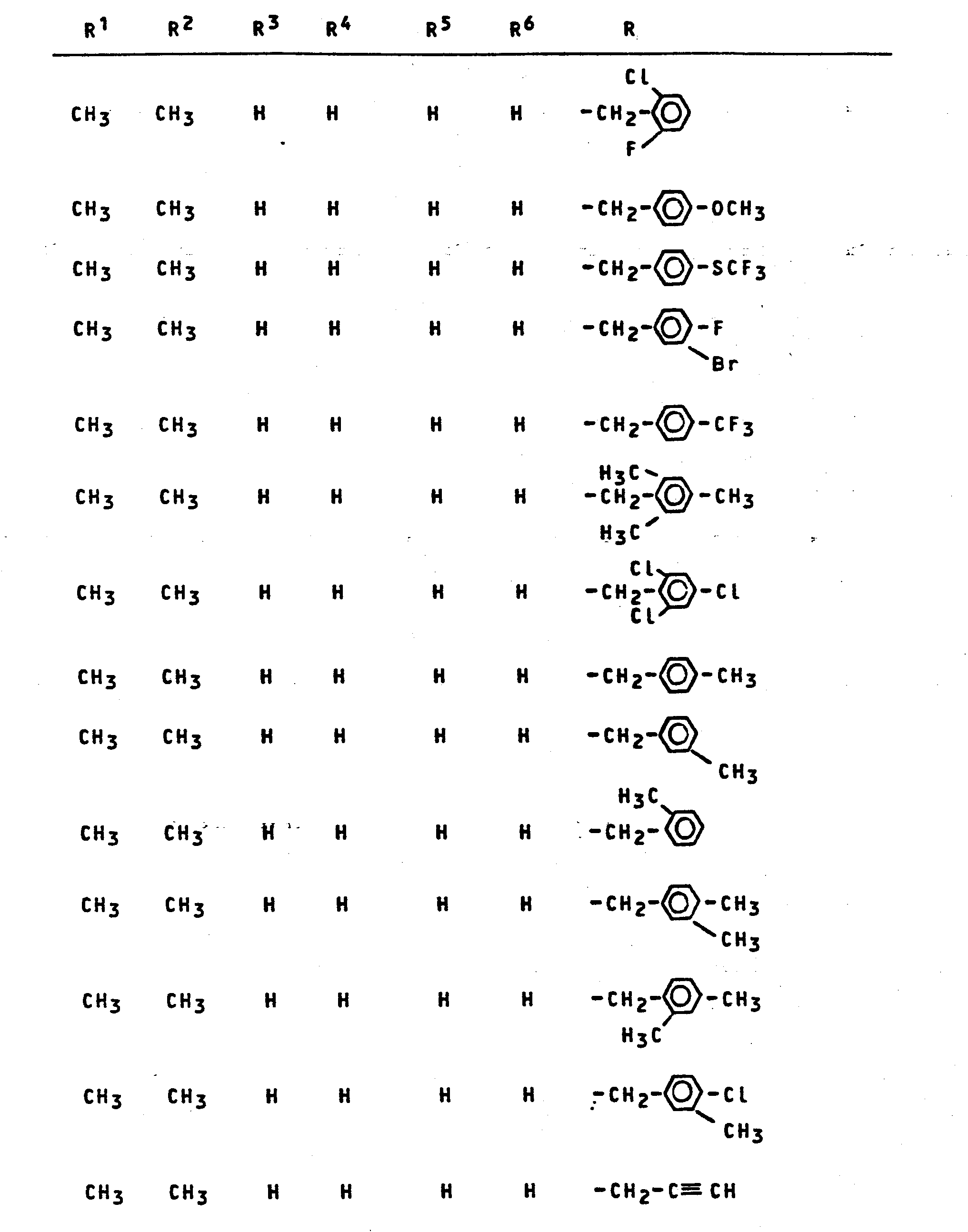 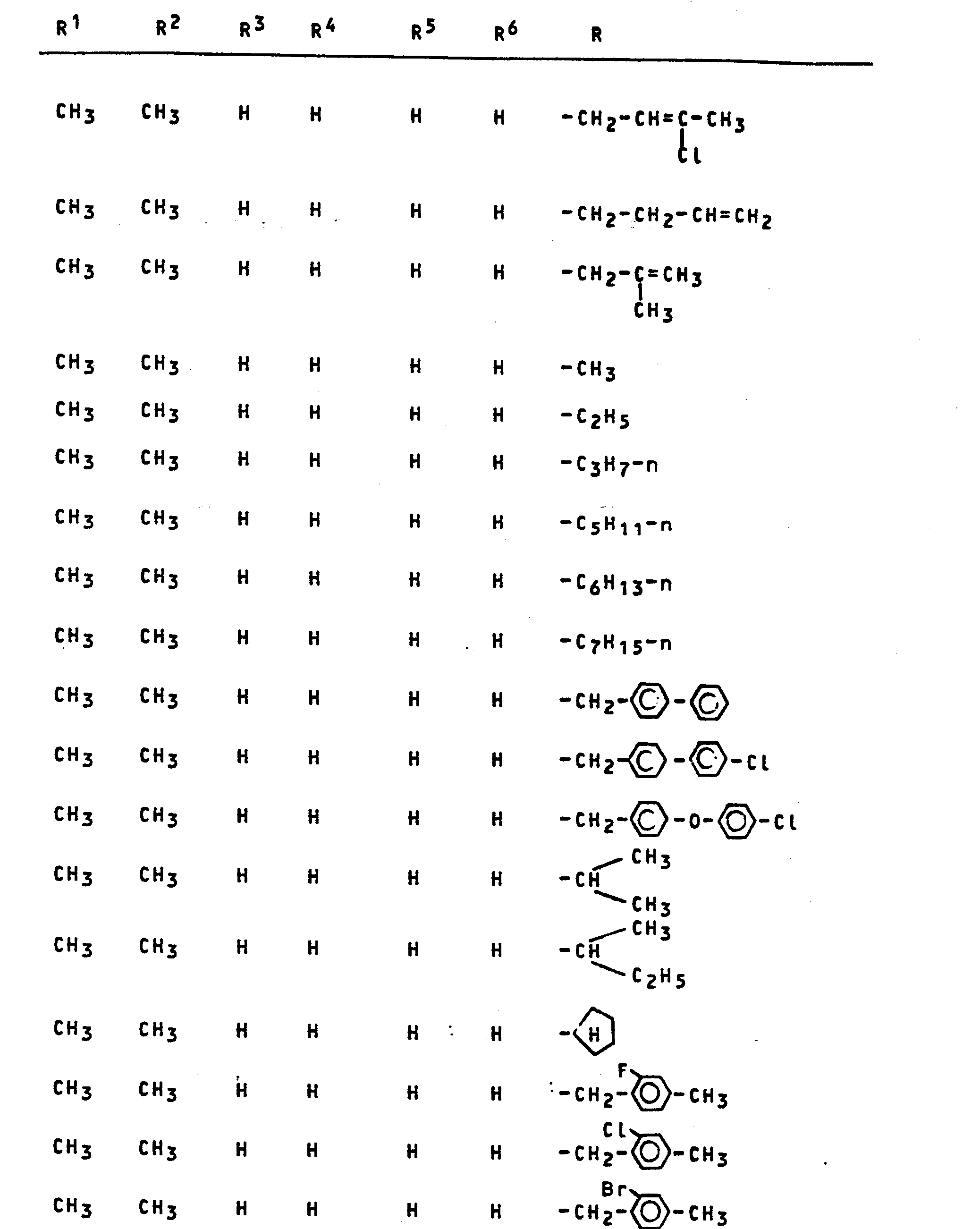 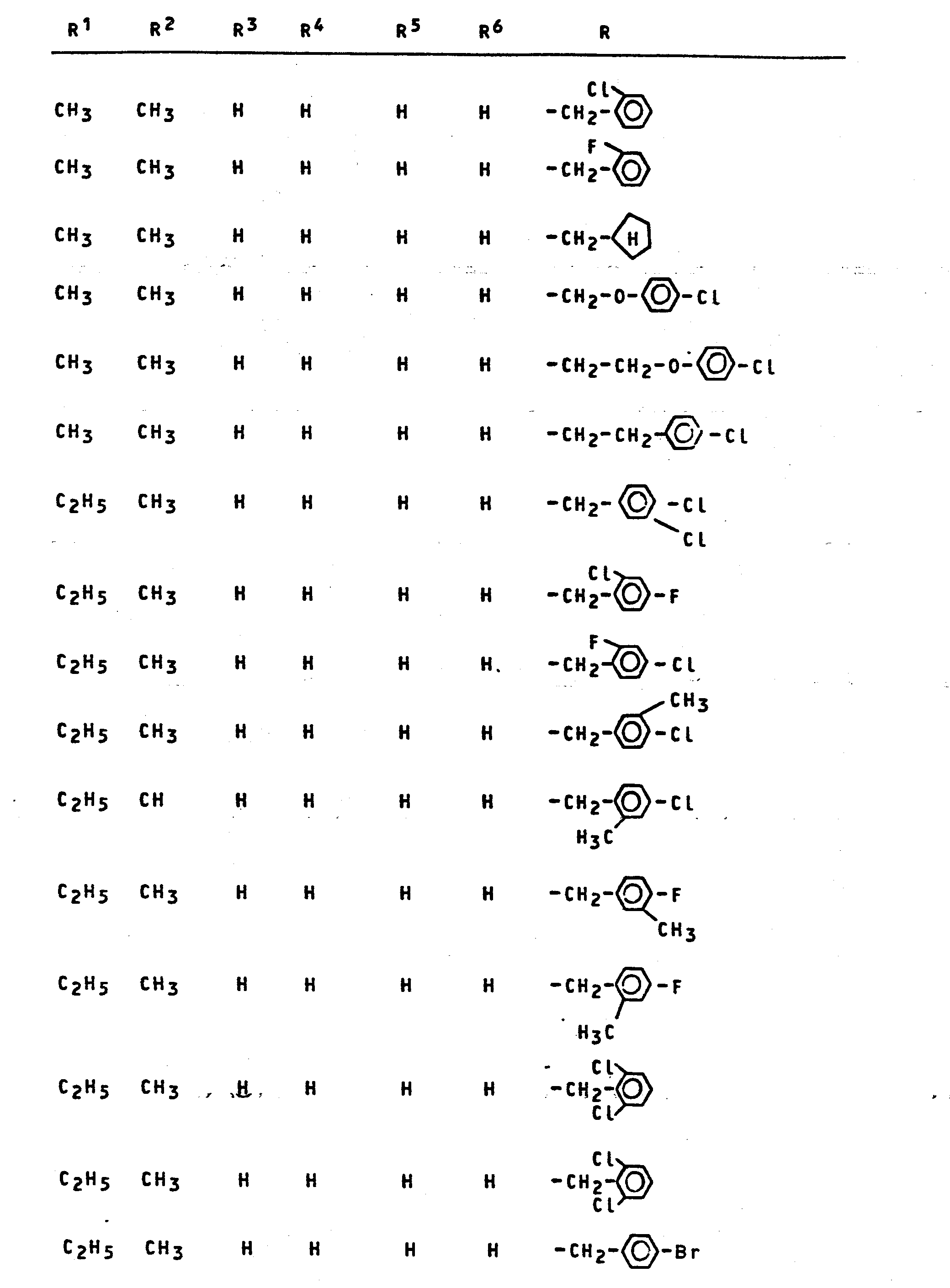 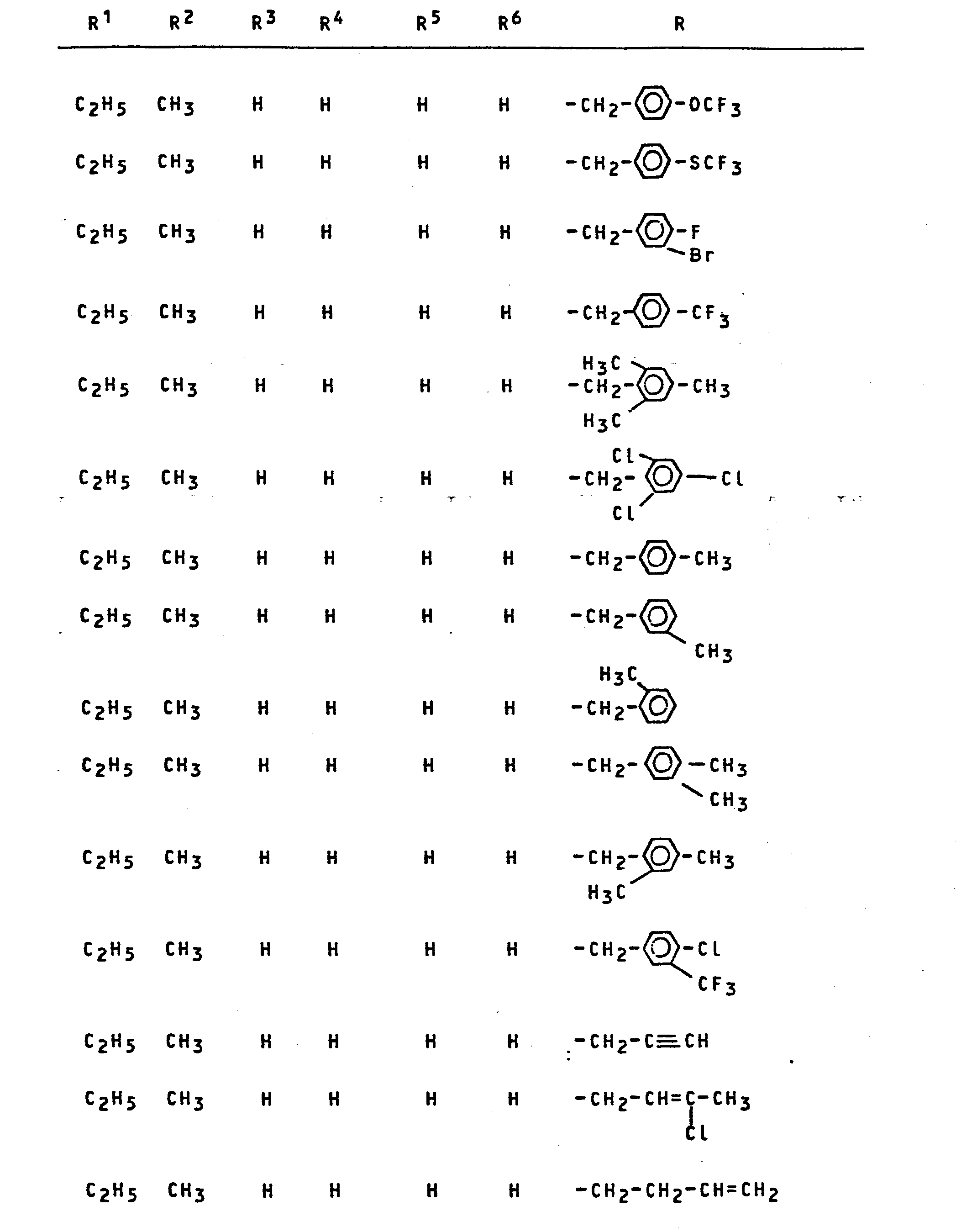 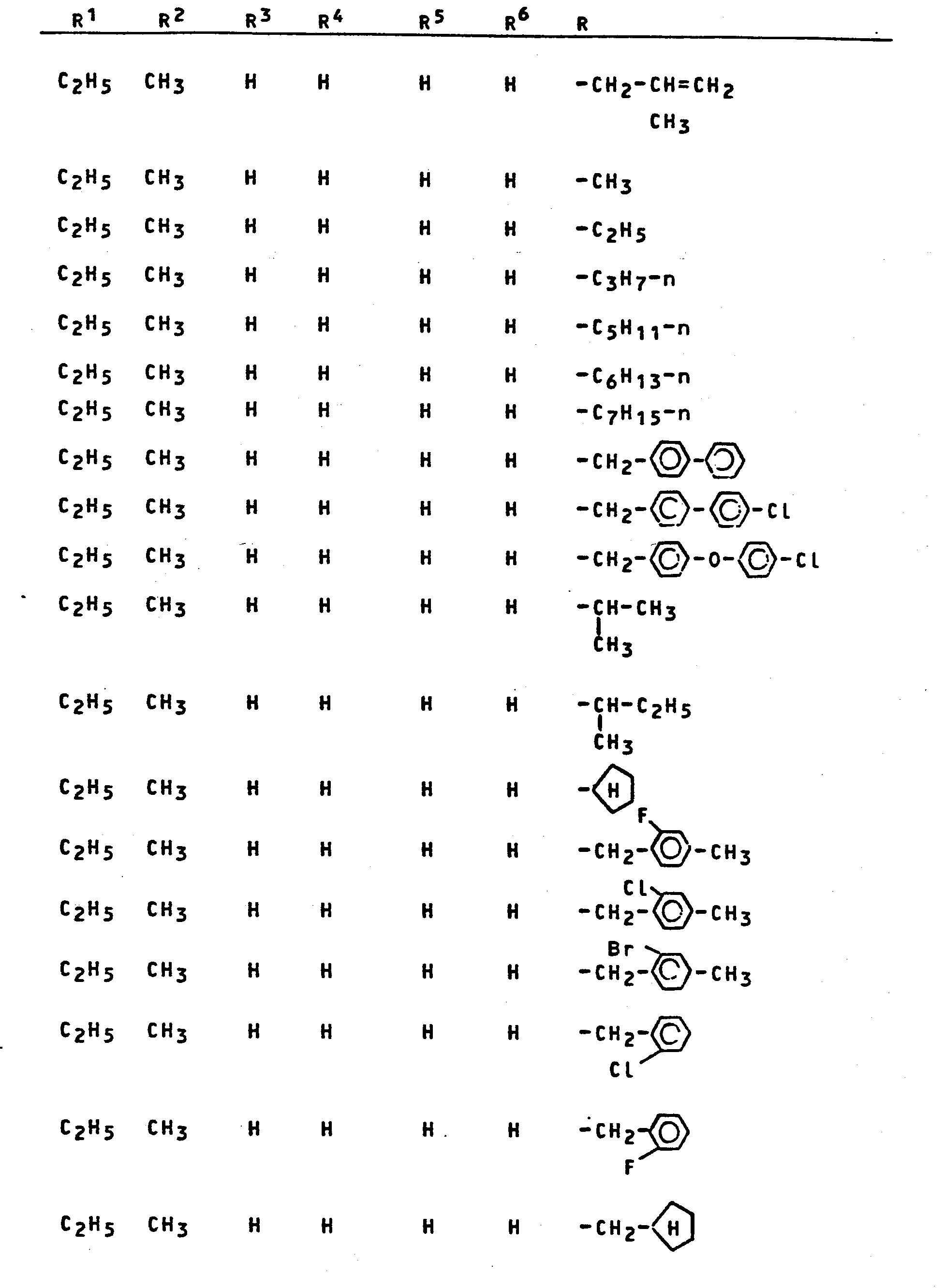 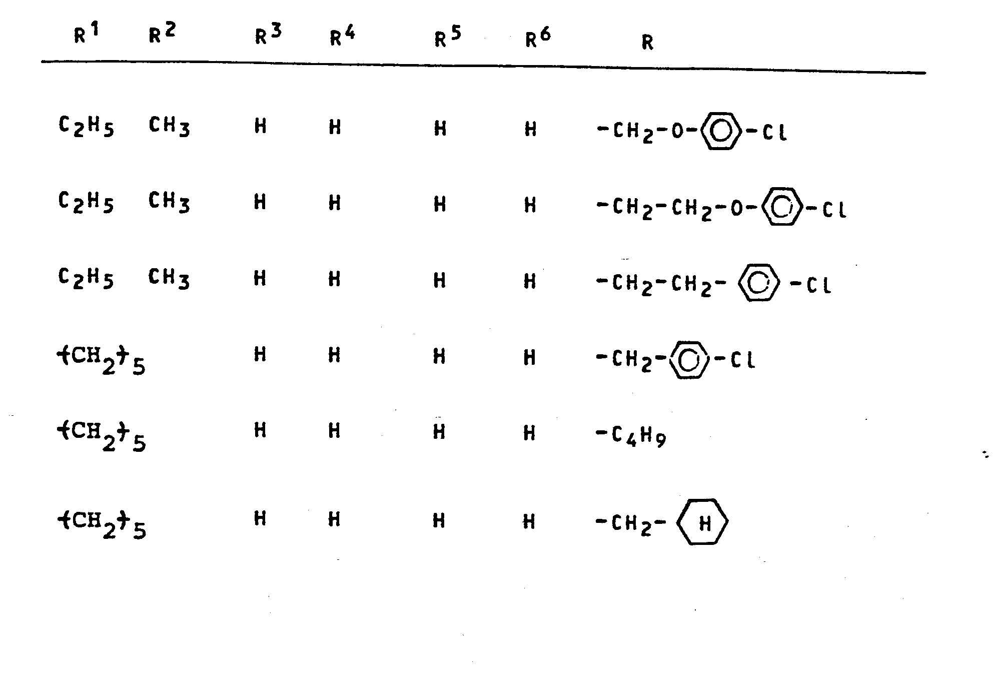 Bevorzugte erfindungsgemäße Verbindungen sind auch Additionsprodukte aus Säuren und denjenigen substituierten Azolyl- tetrahydrofuran-2-yliden-methan-Derivaten der Formel (I), in denen die Substituenten A, R und R¹ bis R⁶ die Bedeutungen haben, die bereits vorzugsweise für diese Substituenten genannt wurden.

Zu den Säuren die addiert werden können, gehören vorzugsweise Halogenwasserstoffsäuren, wie z.B. ChLorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII.Nebengruppe und denjenigen substituierten Azolyl-tetrahydrofuran-2- ytiden-methan-Derivaten der FormeL (I), in denen die Substituenten A, R und R¹ bis R⁶ die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren abteiten, die zu physiologisch verträglichen Additionsprodukten führen. Besonders bevorzugte derartige Säuren sind in diesem Zusammenhang die Halogenwasserstoffsäuren,wie z.B. die Chlorwasserstoffsäure und die Bromwasserstoffsäure, SaLpetersäure und Schwefelsäure. Verwendet man beispielsweise 1-(4-Chlorphenyl)-2-(3,3-dimethyl-2-hydroxy-tetra hydrofuran-2.yl)-2-(1,2,4-triazol-1-yl)-ethan als Ausgangsstoff , so kann der Reaktionsablauf des erfindungsgemäßen Verfahrens durch das folgende FormeLschema wiedergegeben werden: 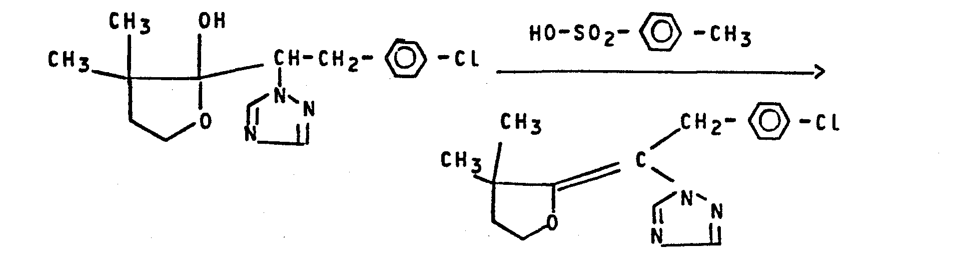 Die bei der Durchführung des erfindungsgemäßen Verfahrens als Ausgangsstoffe zu verwendenden Azolyl-(2-hydroxytetra- hydrofuran-2-yl)-methan-Derivate sind durch die Formel (II) allgemein definiert. In dieser Formel stehen A, R und R¹ bis R⁶ vorzugsweise für die Reste, die bereits im Zusammenhang mit der Beschreibung der erfindungsgemäßen Stoffe der Formel (I) vorzugsweise für diese Substituenten genannt wurden.

Die Azolyl-(2-hydroxy-tetrahydrofuran-2-yl)-methan-Derivate der Formel (II) sind noch nicht bekannt; sie werden erhalten, indem man

### (a) 2-Azolylmethyl-2-hydroxy-tetrahydrofurane der Formel

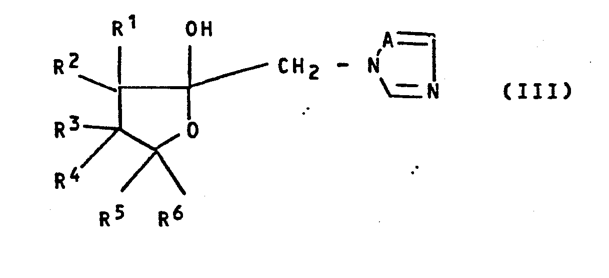

in welcher
A und R¹ bis R⁶ die oben angegebene Bedeutung haben,

mit einem Alkylierungsmittel der Formel (IV) 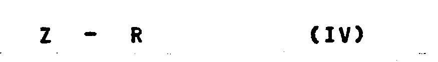 in welcher
R die oben angegebene Bedeutung hat und
Z für eine elektronenanziehende Abgangsgruppierung, wie beispielsweise Halogen, p-Methylphenylsulfonyloxy, die Gruppierung -0-S0₂-OR' oder -NR₃' und andere, steht, worin R' z.B. für einen gegebenenfalls substituierten Kohlenwasserstoffrest steht,

in Gegenwart eines Verdünnungsmittels und in Gegenwart einer Base umsetzt.

Für das Verfahren (a) kommen als Verdünnungsmittel inerte organische Lösungsmittel infrage. Hierzu gehören vorzugsweise aromatische Kohlenwasserstoffe, wie BenzoL, Toluol oder Xylol; halogenierte Kohlenwasserstoffe, wie Methylenchlorid, Tetrachlorkohlenstoff, Chloroform oder Chlorbenzol; Ester, wie Essigester; Formamide, wie Dimethylformamid; sowie Dimethylsulfoxid.

Das Verfahren (a) wird in Gegenwart einer Base durchgeführt. Hierbei können alle üblichen organischen und insbesondere anorganischen Basen eingesetzt werden, wie vorzugsweise Alkalihydroxide oder Alkalicarbonate, beispielsweise seien Natrium- und Kaliumhydroxid genannt.

Die Reaktionstemperaturen können bei der Durchführung des Verfahrens (a) in einem größeren Bereich variiert werden. Im allgemeinen arbeitet man zwischen 0 und 100°C, vorzugsweise zwischen 20 und 100°C.

Bei der Durchführung des Verfahrens (a) setzt man vorzugsweise auf 1 Mol der Verbindung der Formel (III) 1 bis 1,2 Mol Alkylierungsmittel ein. Die Isolierung der Zwischenprodukte der Formel (II) erfolgt in allgemein üblicher Art und Weise.

Das Verfahren (a) kann auch in einem Zweiphasensystem, wie beispielsweise wässrige Natron- oder Kalilauge/Toluol oder Methylenchlorid, gegebenenfalls unter Zusatz von 0,1 bis 1 Mol eines Phasentransfer-Katalysators, wie beispielsweise Ammonium- oder Phosphoniumverbindungen, beispielsweise seien Benzyldodecyldimethyl-ammoniumchlorid und Triethyl-benzylammoniumchlorid genannt, durchgeführt werden.

Die für das Verfahren (a) als Ausgangsstoffe zu verwendenden 2-Azolylmethyl-2-hydroxy-tetrahydrofurane der Formel (III) sind ebenfalls noch nicht bekannt; sie werden er - hatten, indem man

### (b) an 2-Azolylmethylen-tetrahydrofurane der Formel (V)

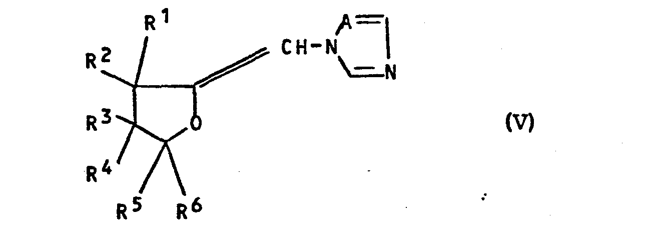

in welcher
A und R¹ bis R6 die oben angegebene Bedeutung haben, in Gegenwart eines Verdünnungsmittels und in Gegenwart einer Säure als Katalysator Wasser addiert.

Für das Verfahren (b) kommen als Verdünnungsmittel mit Wasser mischbare lösungsmittel infrage. Hierzu gehören vorzugsweise Alkohole, wie Methanol oder EthanoL; Ketone, wie Aceton; und Wasser.

Das Verfahren (b) wird in Gegenwart einer Säure als Katalysator durchgeführt. Hierbei können alle üblichen organischen und anorganischen Säuren eingesetzt werden, wie vorzugsweise Schwefelsäure, Salzsäure, Salpetersäure, Methansulfonsäure und p-Toluolsulfonsäure.

Die Reaktionstemperaturen können bei der Durchführung des Verfahrens (b) in einem größeren Bereich variiert werden. Im allgemeinen arbeitet man zwischen 0 und 100°C, vorzugsweise zwischen 20 und 80°C.

Die außerdem für das Verfahren (a) als Ausgangsstoffe zu verwendenden Alkylierungsmittel der Formel (IV) sind allgemein bekannte Verbindungen der organischen Chemie.

Die für das Verfahren (b) als Ausgangsstoffe zu verwendenden 2-Azolylmethylenttetrahydrofurane der formel (V) sind teilweise bekannt (vergleiche DE-OS 32 04 795). Noch nicht bekannt sind die 2-Azolylmethylen-tetrahydrofurane der Formel (Va). 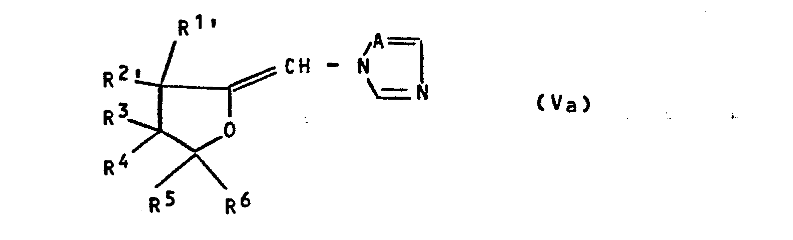 in welcher
A und R³ bis R⁶ die oben angegebene Bedeutung haben und
R¹' und R²' für die oben genannten Bedeutungen von R¹ bzw. R² stehen, wobei die beiden Reste nicht gleichzeitig für MethyL stehen, wenn R³ bis R⁶ gleichzeitig für Wasserstoff stehen.

Die 2-Azolylmethylen-tetrahydrofurane der FormeL (V) bzw. (Va) werden erhalten, indem man

### (c) Hatogenketone der Formel (VI)

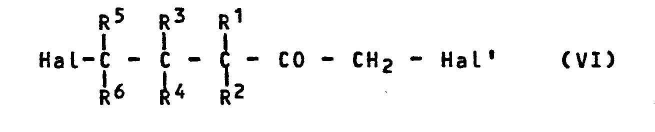

in welcher
R¹ bis R⁶ die oben angegebene Beduetung haben und
Hal und Hal' für Halogen, vorzugsweise ChLor oder Brom stehen,

mit Azolen der Formet (VII) 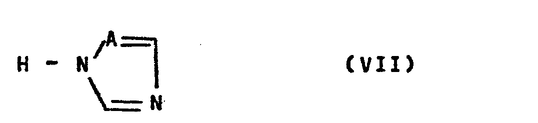 in welcher
A die oben angegebene Bedeutung hat,

in Gegenwart eines Verdünnungsmittels und in Gegenwart eines Säurebindemittels umsetzt.

Für das Verfahren (c) kommen als Verdünnungsmittel inerte organische Lösungsmittel infrage. Hierzu gehören vorzugsweise Ketone, wie Diethylketon und insbesondere Aceton und Methylethylketon; Nitrite, wie Propionitril, insbesondere Acetonitril; Alkohole, wie Ethanol oder Isopropanol; Ether, wie Tetrahydrofuran oder Dioxan; aromatische Kohlenwasserstoffe, wie Toluol, Benzol oder ChLorbenzoL; Formamide, wie insbesondere Dimethylformamid; und halogenierte KohLenwasserstoffe.

Das Verfahren (c) wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-DimethyLbenzytamin, weiterhin Pyridin und Diazabicyctooctan.

Vorzugsweise verwendet man einen entsprechenden Ueberschuß an Azol.

Die Reaktionstemperaturen können bei der Durchführung des Verfahrens (c) in einem größeren Bereich variiert werden. Im altgemeinen arbeitet man zwischen etwa 20 bis 150°C, vorzugsweise bei 60 bis 120°C.

Bei der Durchführung des Verfahrens (c) setzt man auf 1 Mol der Verbindungen der Formel (VI) vorzugsweise 1 bis 4 Mol Azol der Formel (VII) und 1 bis 4 Mol Säurebinder ein. Die Isolierung der Verbindungen der Formel (V) bzw. (Va) erfolgt in üblicher Art und Weise.

Die für das Verfahren (c) als Ausgangsstoffe zu verwendenden Halogenketone der Formel (VI) sind teilweise bekannt (vergleiche DE-OS 32 04 788). Noch nicht bekannt sind die Halogenketone der Formel (VIa) 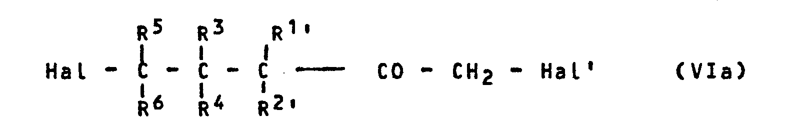 in welcher
Hal, Hal', R³ bis _{R}⁶ und R¹' und R²¹ die oben angegebene Bedeutung haben.

Die Halogenketone der Formel (VI) bzw. (VIa) werden erhatten, indem man

### (d) 2-Chlormethylen-tetrahydrofurane der Formel (VIII)

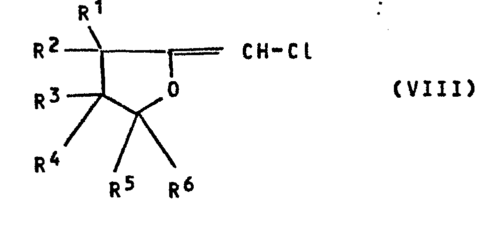

in welcher
R¹ bis R⁶ die oben angegebene Bedeutung haben,

mit aciden Verbindungen der Formel (IX) 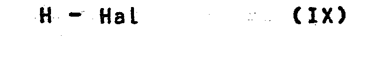 in welcher
Hal die oben angegebene Bedeutung hat,

gegebenenfalls in Gegenwart eines Verdünnunsgmittets umsetzt.

Für das Verfahren (d) kommen als Verdünnungsmittel inerte organische Lösungsmittel infrage. Hierzu gehören vorzugsweise aromatische Kohlenwasserstoffe, wie Toluol, Benzol oder Chlorbenzol; Formamide, wie Dimethylformamid; Nitrile, wie Acetonitril; und halogenierte Kohlenwasserstoffe, wie Methylenchlorid.

Die Reaktionstemperaturen können bei der Durchführung des Verfahrens (d) in einem größeren Bereich variiert werden. Im allgemeinen arbeitet man zwischen 0 und 150°C;] vorzugsweise bei 10 bis 120°C.

Bestimmte Hatogenketone der Formel (VI) bzw. (VIa) können auch erhalten werden, indem man entsprechend substituierte Ketone bzw. ungesättigte Ketone in üblicher Weise mit Halogenierungsmitteln, wie beispielsweise Brom, Brom- wasserstoff oder Sulfurylchlorid umsetzt (vergleiche hierzu auch die Hersteltungsbeispiele).

Die außerdem für das Verfahren (c) als Ausgangsstoffe zu verwendenden Azole der Formel (VII) sind allgemein bekannte Verbindungen der organischen Chemie.

Die für das Verfahren (d) als Ausgangsstoffe zu verwendenden 2-Chlormethylen-tetrahydrofurane der Formel (VIII) sind teitweise bekannt (vergleiche DE-OS 32 04 692). Noch nicht bekannt sind die 2-Chlormethylen-tetrah^{y}drofurane der Formel (VIIIa) 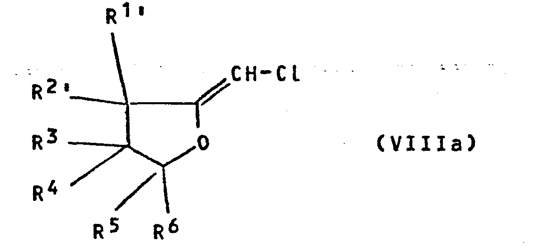 in welcher
R¹' und R²' sowie R³bis R⁶ die oben angegebene Bedeutung haben.

Die 2-Chlormethyten-tetrahydrofurane der Formel (VIII) bzw. (IIIIa) werden erhalten, indem man z.B.

### e) 1,1,5-Trichlor-penten-Derivate der Formel (X)

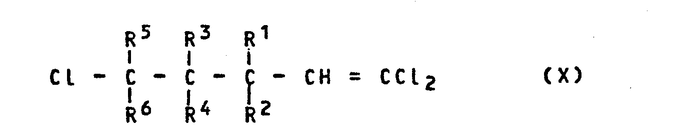

in welcher
R¹ bis R⁶ lie oben angegebene Bedeutung haben,

oder 1,1,1,5-etrachlor-pentan-Derivate der Formel (XI) 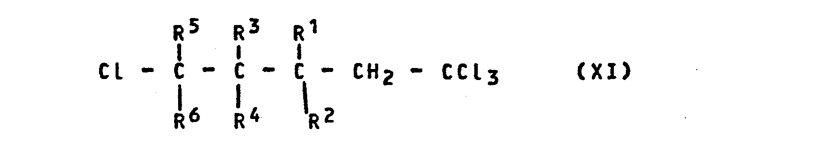 in welcher
R¹ bis R⁶ die oben angegebene Bedeutung haben,

mit Carboxytaten, wie insbesondere wasserfreiem Natriumacetat, und mit Basen, wie insbesondere Alkalialkoholaten, in Gegenwart eines Verdünnungsmittels umsetzt.

Für das Verfahren (e) kommen als Verdünnungsmittel inerte organische Lösungsmittel infrage. Hierzu gehören vorzugsweise Formamide, wie Dimethylformamid; Nitrite, wie Acetonitril; und halogenierte Kohlenwasserstoffe.

Die Reaktionstemperaturen können bei der Durchführung des Verfahrens (e) in einem größeren Bereich variiert werden. Im allgemeinen arbeitet man zwischen 20 und 150°C, vorzugsweise bei der Siedetemperatur des verwendeten Lösungsmittels.

Bei der Durchführung des Verfahrens (e) setzt man auf 1 Mol der Verbindungen der FormeLn (X) bzw. (XI) vorzugsweise 1 bis 2 Mol an Carboxylat und 1 bis 2 Mol an Base ein. Die Isolierung der gewünschten Produkte erfolgt in üblicher Art und Weise.

Die außerdem für das erfindungsgemäße Verfahren (d) als Ausgangsstoffe zu verwendenden aciden Verbindungen der Formel (IX) sind allgemein bekannte Verbindungen der organischen Chemie.

Die für das Verfahren (e) als Ausgangsstoffe zu verwendenden Verbindungen der Formel (X) bzw. (XI) sind bekannte Verbindungen der organischen Chemie bzw. können sie in bekannter Art und Weise erhalten werden.

Für die erfindungsgemäße Umsetzung kommen als Verdünnungsmittel inerte organische Lösungsmittel infrage. Hierzu gehören vorzugsweise aromatische Kohlenwasserstoffe, wie Toluol, Benzol, Xylol, Chlorbenzol oder Dichlorbenzol; und halogenierte aliphatische Kohlenwasserstoffe, wie Dichlorethan.

Die erfindungsgemäße Umsetzung wird in Gegenwart eines sauren Katalysators vorgenommen. Hierzu gehören vorzugsweise aliphatische und aromatische sulfonsäuren, wie p-Toluolsulfonsäure oder Methansulfonsäure, die gegebenenfalls auch in polymer gebundener Form vorliegen können.

Die Reaktionstemperaturen können bei der erfindungsgemäßen Umsetzung in einem größeren Bereich variiert werden. Im allgemeinen arbeitet man zwischen etwa 50 und 150°C, vorzugsweise bei 80 bis 120°C.

Bei der Durchführung der erfindungsgemäßen Umsetzung setzt man auf 1 Mol der Verbindungen der Formel (II) katalytische Mengen an saurem Katalysator ein. Die Isolierung der Verbindungen der Formel (I) erfolgt in üblicher Art und Weise. Die Säureadditionssalze der Verbindungen der formel (I) können in einfacher Weise nach üblichen Salzbildungsmethoden, z.ß. durch Lösen einer Verbindung der Formel (I) in einem geeigneten inerten Lösungsmittel und Hinzufügen der Säure, z.B. ChLorwasserstoffsäure, erhalten werden und gegebenenfalls durch Waschen mit einem organischen lösungsmittel gereinigt werden.

Die Metallsalz-Komplexe von Verbindungen der Formel (I) können in einfacher Weise nach üblichen Verfahren erhalten werden, so z.B. durch Lösen des metallsalzes in Alkohol, z.B. Ethanol und Hinzufügen zu Verbindungen der Formel (I). Man kann Metallsalz-Komplexe in bekannter Weise, z.B. durch Abfiltrieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von PTlanz- und Saatgut, und des Bodens.

Als Pflanzenschutzmittel können die erfindungsgemäßen Wirkstoffe mit besonders gutem Erfolg zur Bekämpfung von Sphaerotheca-Arten, wie gegen den Erreger des echten Gurkenmehttaus (Sphaerotheca fuliginea); von Botrytis-Arten, wie gegen den Erreger des Grauschimmels (Botrytis cinerea); von Getreidekrankheiten, wie Erysiphe graminis Roste, Septoria, Cochliobolus sativus,Pyrenophora teres oder Pyrenophora graminea; sowie von Reiskrankheiten, wie Pyricularia oryzae und Pellicularia sasakii, eingesetzt werden. Außerdem zeigen die erfindungsgemäßen Stoffe ein breites und gutes fungizides in vitro-Wirkungsspektrum

Hervorzuheben ist, daß die erfindungsgemäßen Stoffe nicht nur eine protektive Wirkung entfalten, sondern teilweise auch systemisch sind. So gelingt es,Pftanzen gegen Pilzbefall zu schützen, wenn man die Wirkstoffe über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Spritzpulvdr, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungpn.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls.unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln:

Die Wirkstoffe können als solche, in Form ihrer Formulierungen oder der daraus durch weiteres Verdünnen be--reiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise ₀,₀1 bis 10 g, benötigt.

Bei Behandlung des Bodens sind.Wirkstoffkonzentrationen von 0,00001 bis 0,1 Gew.-%, vorzugsweise von 0,0001 bis '0,02 Gew.-%, am Wirkungsort erforderlich.

### Herstellungsbeispiele:

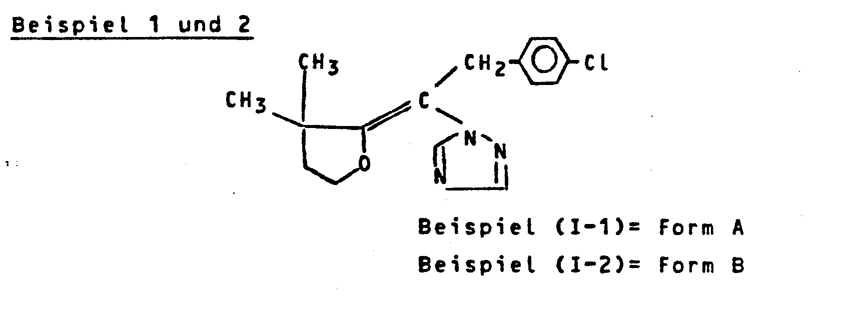

(FormA und Form B = die beiden möglichen geometrischen Isomeren)

5g (0,0155 Mol) 1-(4-Chlorphenyl)-2-(3,3-dimethyl-2-hydroxy- tetrahydrofuran-2-yl)-2-(1,2,4-triazol-1-yl)-ethan werden in 100 ml Toluol gelöst, mit 0,5g p-Toluolsulfonsäure versetzt und 2 Stunden unter Rückfluß am Wasserabscheider erhitzt. Das Reaktionsgemisch wird abgekühlt, mit Wasser gewaschen, über Natriumsulfat getrocknet und im Vakuum eingeengt. Der Rückstand wird säulenchromatographisch (Kieselgel;Essigester/Cyclohexan = 3:1) gereinigt. Man erhält 2,8g (59,6 % der Theorie) 1-(4-Chlorphenyl)-2-(3,3-dimethyl-tetrahydrofuran-2-yliden)-2-(1,2,4-triazot-1-yt)-ethan als Form A vom SchmeLzpunkt 89°C und 0,4g (8,5 % der Theorie) 1-(4-Chlorphenyl)-2-(3,3-dimethyl-tetrahydrofuran-2-yliden)-2-(1,2,4-triazol-1-yl)-ethan als Form B vom Schmelzpunkt 175-177°C.

### Herstellung des Ausgangsproduktes

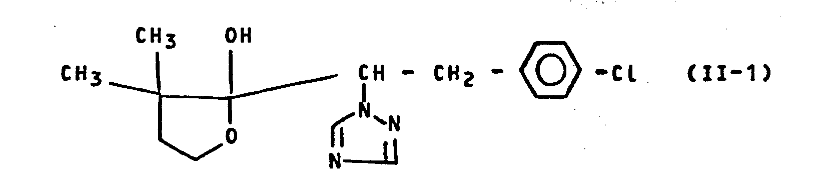

10,3g (0,052 Mol) 3,3-Dimethyt-2-hydroxy-2-(1,2,4-triazol-1-yl-methyt)-tetrahydrofuran, 2,9g (0,052 Mol) Kaliumhydroxid und 8,4g (0,052 Mol) 4-Chtorbenzylchlorid werden in einem Gemisch aus 100 ml Dimethylsulfoxid und 6 ml Wasser 2 Stunden bei 30°C gerührt. Danach gibt man die Reaktionslösung auf Wasser und extrahiert mit Essigester. Die organische Phase wird über Natriumsulfat getrocknet und eingeengt. Der Rückstand kristallisiert nach Verreiben in n-Hexan. Man erhält 6,6g (39,5 % der Theorie) 1-(4-Chlorphenyl)-2-(3,3-dimethyl-2-hydroxy-tetrahydrofuran-2-yl)-2-(1,2,4-triazol-1-yl)-ethan vom Schmelzpunkt 98°C. 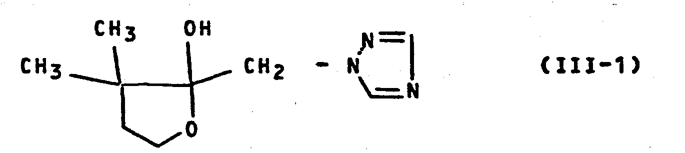 30g (0,17 Mol) (3,3-Dimethyl-tetrahydrofuran-2-yliden)-(1,2,4-triazol-1-yl)-methan und 100g konzentrierte Salzsäure werden in 300 ml Methanol 4 Stunden bei Raumtemperatur gerührt. Das Reaktionsgemisch wird im Vakuum eingeengt, der Rückstand in Wasser aufgenommen, mit verdünnter Natronlauge auf einen pH-Wert von 7 bis 8 gestellt und mit Methylenchlorid extrahiert. Die organische Phase wird mit Wasser gewaschen, über Natriumsulfat getrocknet und im Vakuum eingeengt. Man erhält 30,7g (93 X der Theorie) 3,3-Dimethyl-2-hydroxy-2-(1,2,4-triazol-1-yl-methyl)-tetrahydrofuran vom Schmelzpunkt 93-95°C. 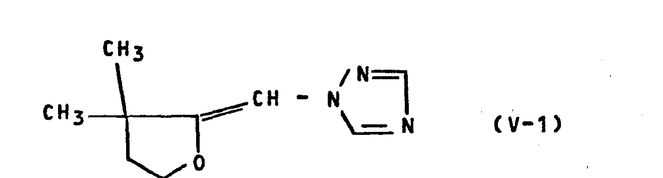 85g (1,23 Mol) 1,2,4-Triazol und 464g (33,6 Mol) Kaliumcarbonat werden in 2,5 l Aceton vorgelegt. Bei Raumtemperatur Läßt man ohne KühLung unter Rühren 220g (1,12 Mol) 1,5-Dichlor-3,3-dimethyl-2-pentanon zutropfen. Nach Beendigung der Zugabe Läßt man 2 Stunden bei Rückflußtemperatur nachrühren.

Das Reaktionsgemisch wird abgekühlt, über eine Nutsche abgesaugt und die Mutterlauge im Vakuum eingeengt. Der Rückstand wird in Methylenchloridaufgenommen, mit Wasser gewaschen, über Natriumsulfat getrocknet und eingeengt. Der Rückstand wird in PetroLether verrührt, abgesaugt und im Vakuum bei 50°C getrocknet. Man erhält 145,3g (56,5 X der Theorie) (3,3-Dimethyl-tetrahydrofuran-2-yliden)-(1,2,4-triazol-1-yl)-methan vom SchmeLzpunkt 47°C. 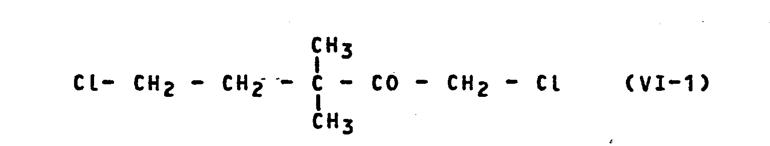 In 476g (3,25 Mol) 2-Chlormethylen-3,3-dimethyltetrahydro- furan wird unter Eiskühlung ein starker Chlorwasserstoffgasstrom aus einer Bombe eingeleitet. Das Gas wird Vollständig absorbiert und die Innentemperatur steigt bis auf 30°C. Nach vollständiger Sättigung mit Chlorwasserstoff wird das Reaktionsgemisch 2 Stunden bei Raumtemperatur nachgerührt. Ueberschüssiger Chlorwasserstoff wird zunächst in die Wasserstrahlpuape gezogen, dann wird bei gutem Vakuum destilliert. Man erhält 531 g (90 X der Theorie) 1,5-Dichlor-3,3-dimethyl-2-pentanon vom Siedepunkt 85-90°C/0,3mbar. 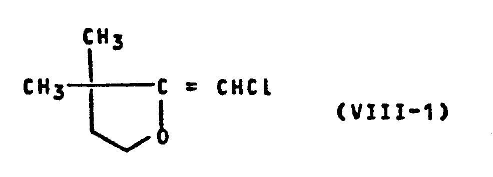 806g (4 Mol) 1,1,5-Trichtor-3,3-dimethyt-1-penten werden mit 360 g (4,4 Mol) wasserfreiem Natriumacetatin 1 L Dimethylformamid 6 Stunden unter Rückfluß erhitzt. Nach Abkühlen auf ca. 100°C tropft man 1,6 L (8 Mot) 30 %ige Natriummethylatlösung in Methanol ein und erhitzt weitere 4 Stunden unter Rückfluß. Die kalte Lösung wird in Wasser gegossen udn mit MethyLenchLorid mehrfach extrahiert. Nach Trocknen der Lösung und Abdestillieren des Lösungsmittels bleiben 654 g Produkt zurück, das über eine KoLonne frakioniert wird. Man erhält 522g (89 X der Theorie) 2-Chlormethylen-3,3-dimethyl- tetrahydrofuran vom Siedepunkt 84-87°C/20 mbar.

In entsprechender Weise und gemäß den angegebenen Verfahrensbedingungen können die folgenden Endprodukte der allgemeinen Formel (I)

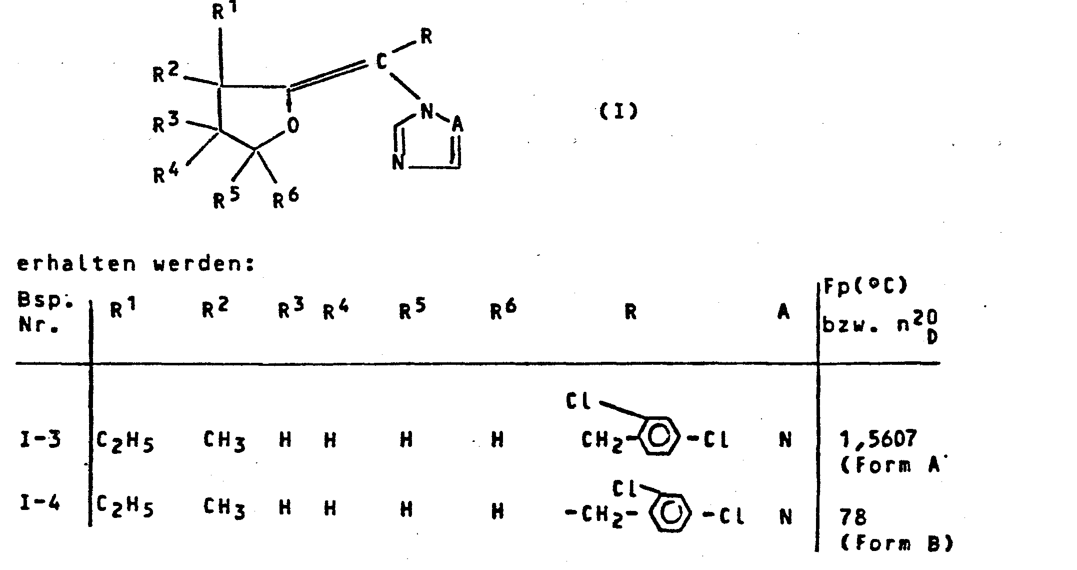

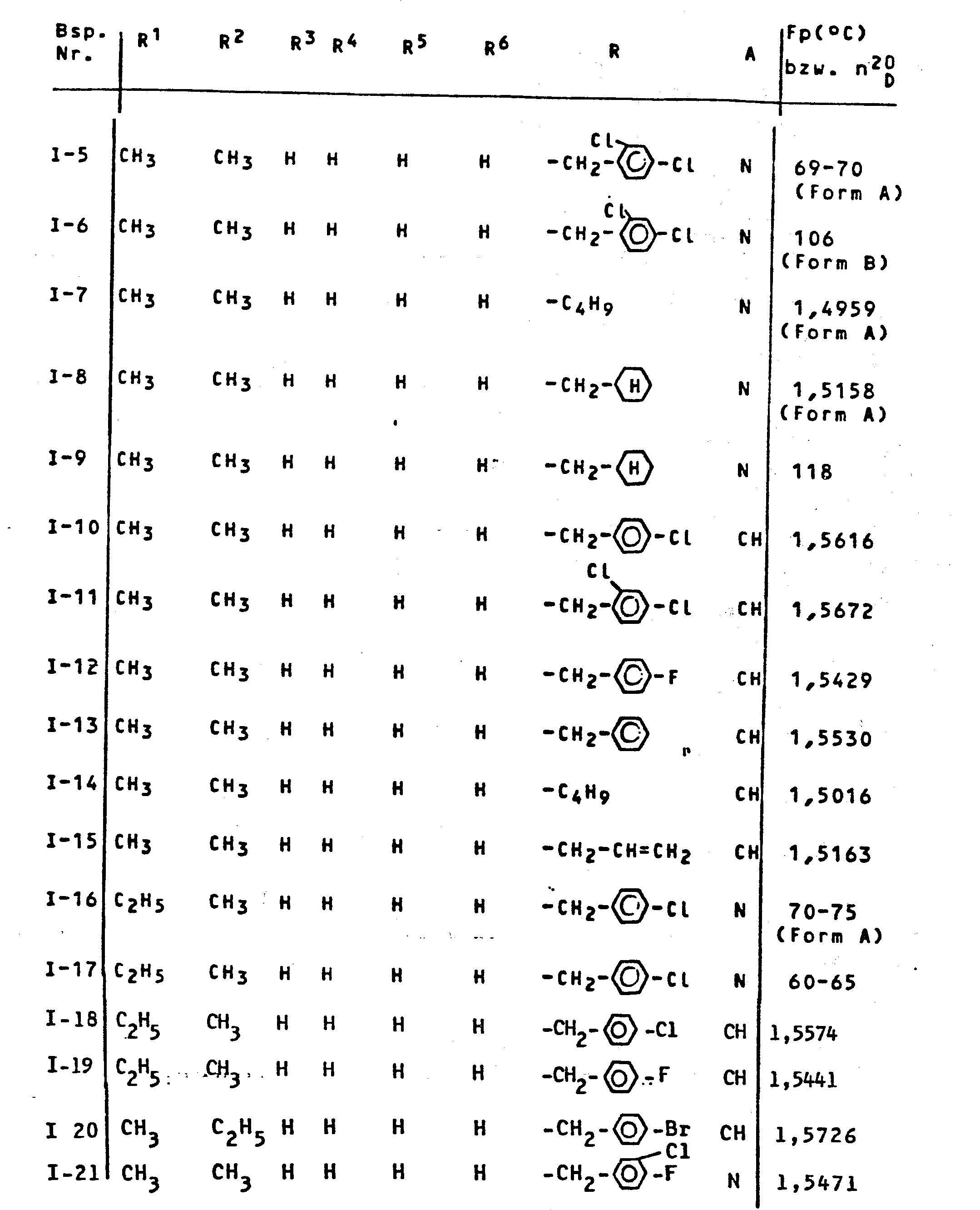

Form A und Form B die beiden möglichen geometrischen Isomeren Entsprechend BeispieL 1 und gemäß den angegebenen Verfahrensbedingungen können die folgenden Ausgangsprodukte der Formel (II)

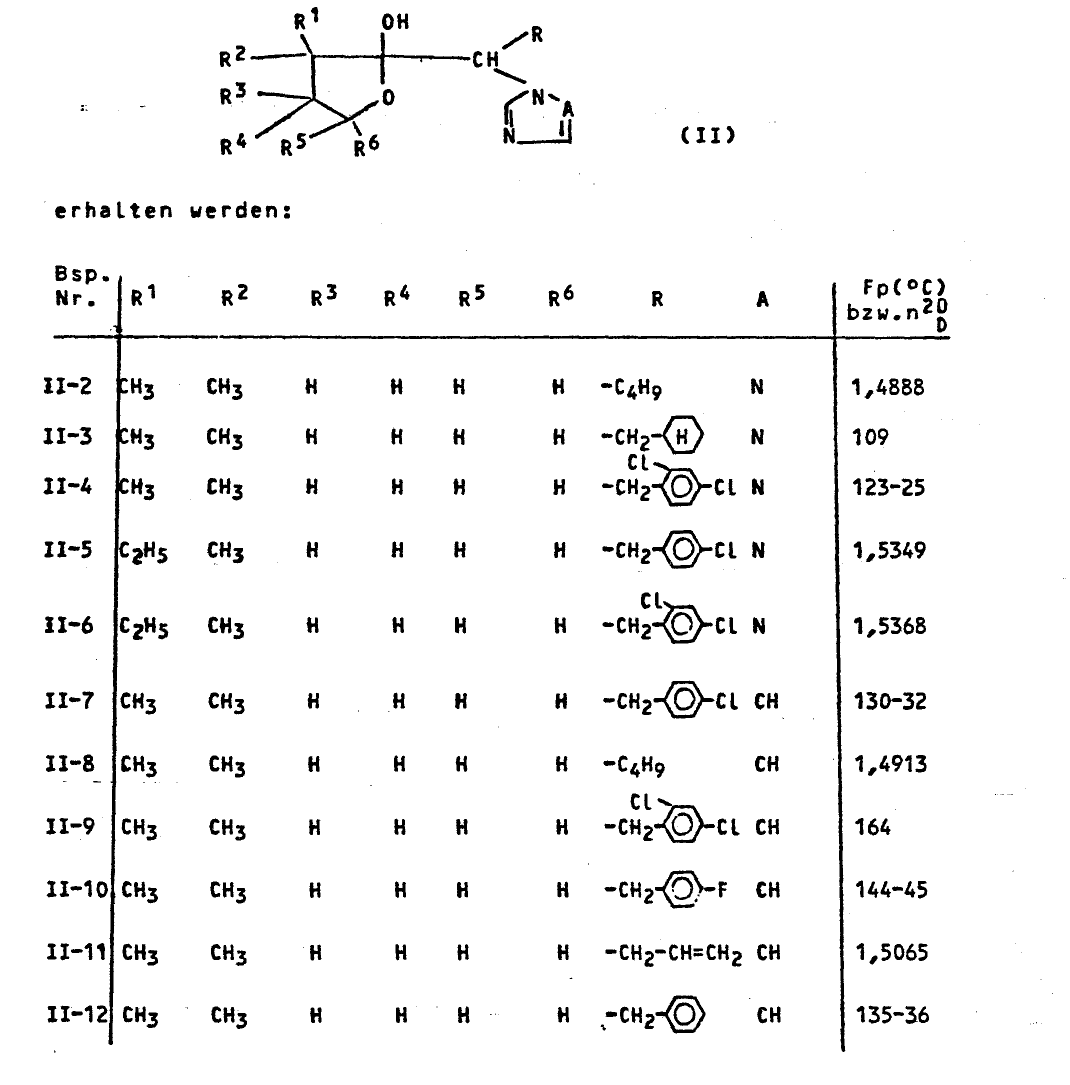

Entsprechend Beispiel 1 und gemäß den angegebenen Verfahrensbedingungen können die folgenden Ausgangsprodukte der Formel (III)

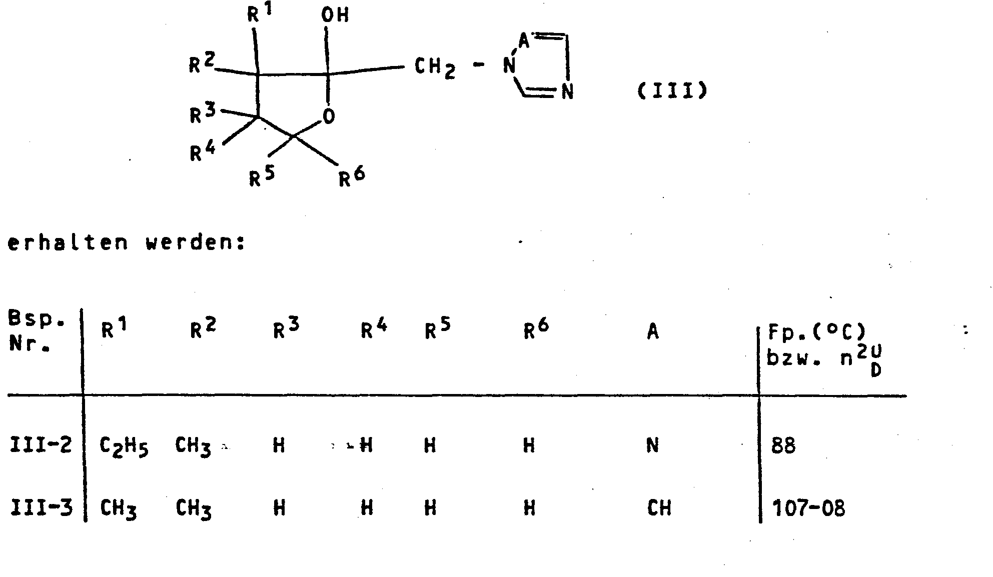

Entsprechend Beispiel 1 und gemäß den angegebenen Verfahrensbedingungen können die folgenden Ausgangsprodukte der Formel (Va)

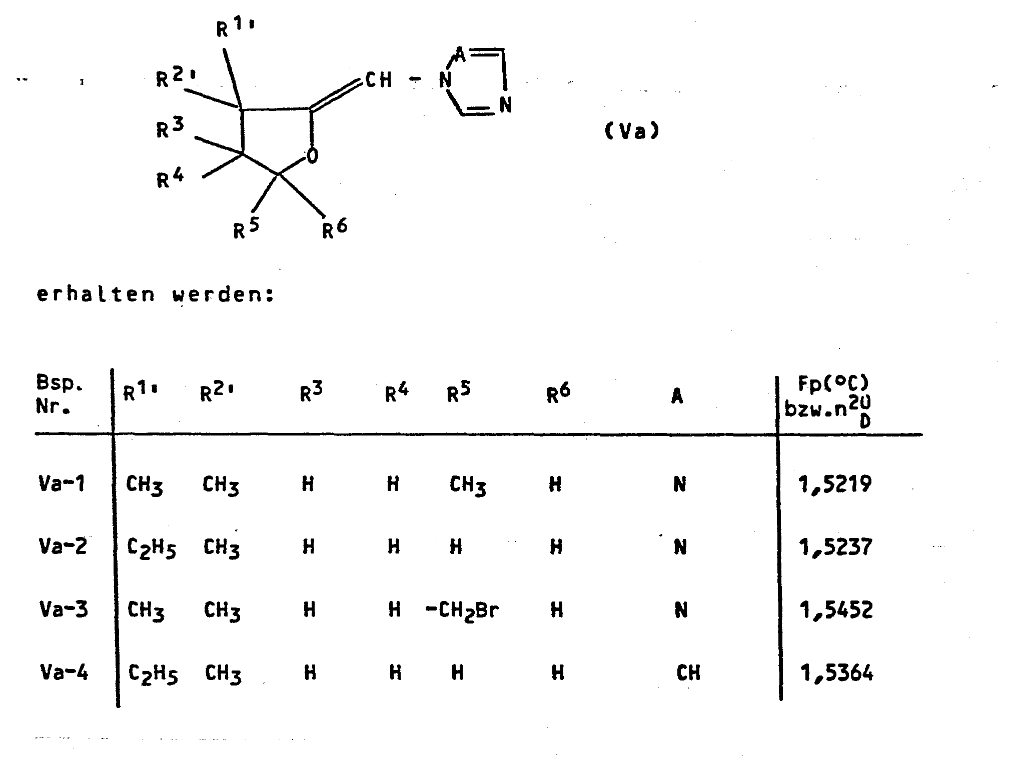

Die folgenden Beispiele belegen weitere Darstellungsmöglichkeiten von Ausgangsprodukten der Formel (VI) bzw. (VIa):

### Beispiel a

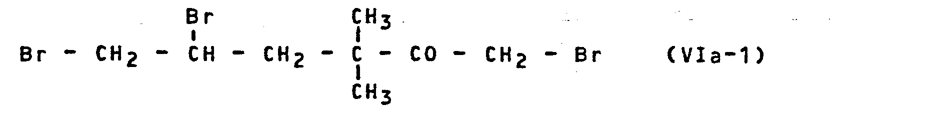

67,2g (0,533 MoL) 3,3-Dimethyl-5-hexen-2-on in 500 ml Chloroform werden bei Raumtemperatur tropfenweise mit 170,7g (1,066 Mol) Brom versetzt. Man läßt 30 Minuten nachrühren, wäscht mit Wasser, trocknet über Natriumsulfat und engt im Vakuum ein. Man erhält 188g (96,5 X der Theorie) 3,3-Dimethyl-1,5,b-tribrom-2-hexanon vom BrechungsindeX n²⁰_{D} =1,5387. 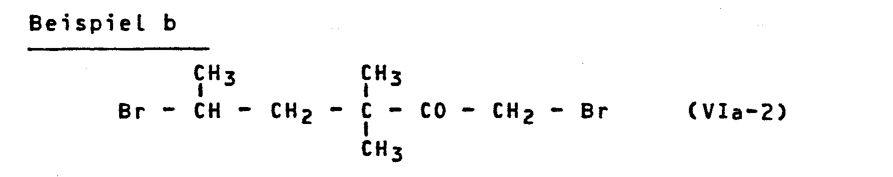 27g (0,13 Mol) 5-Brom-3,3-dimethyl-2-hexanon in 50 ml Chloroform werden mit 20,9g (0,13 Mol) Brom tropfenweise bei Raumtemperatur versetzt. Man läßt 30 Minuten bei Raumtemperatur nachrühren, wäscht mit Wasser, trocknet über Natriumsulfat und engt im Vakuum ein. Man erhält 36,9g (99% der Theorie) 1,5-Dibrom-3,3-dimethyl-2-hexanon vom Brechungsindex n²⁰_{D} = 1,5015.

### Herstellung des Vorproduktes

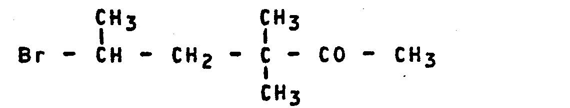

18,9g (0,15 Mol) 3,3-Dimethyl-5-hexen-2-on und 1,6g (0,0054 Mol) Eisen-III-bromid werden in 150 ml Chloroform gelöst. Bei 0°C werden innerhalb von 90 Minuten 0,16 Mol Bromwasserstoffgas eingeleitet. Man läßt 2 Stunden bei Raumtemperatur nachrühren, die organische Phase wird abgetrennt, mit Wasser gewaschen, über Natriumsulfat getrocknet und im Vakuum eingeengt. Man erhält 27g (87 X der Theorie) 5-Brom-3,3-dimethyl-2-hexanon vom Brechungsindex n²⁰_{D} = 1,4639.

### Verwendungsbeispiele

In den nachfolgenden BeispieLen werden die nachstehend angegebenen Verbindungen als Vergleichssubstanzen eingesetzt:

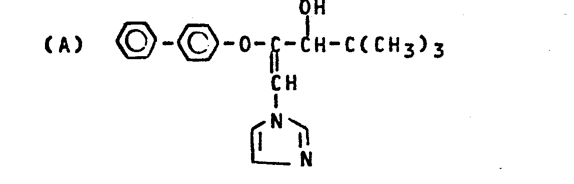

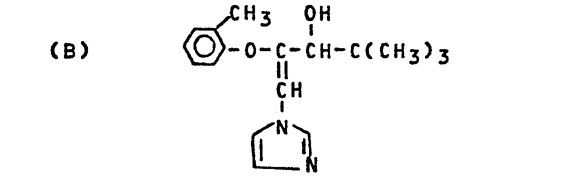

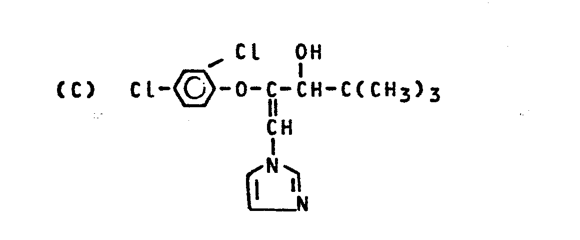

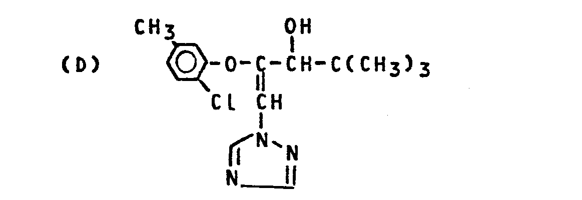

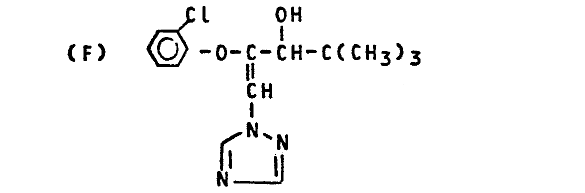

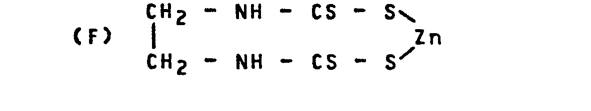

### Beispiel A

Botrytis-Test ( Bohne )/protektiv
Lösungsmittel:4,1 Gewichtsteile Aceton
Emulgator: 0,3 Gewichtsteile Alkylarylpolyglkolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß der Herstellungsbeispiele: 1, 6, 10, 16 und 17.

### Beispiel _{B}

Sphaerotheca-Test (Gurke) / protektiv
Lösungsmittel: 4,7Gewichtsteile Aceton
Emulgator: 0,3 Gewichtsteile Alkylarylpotyglkolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele: 5 und 6.

### Beispiel C

Pyricularia-Test (Reis) /protektiv
Lösungsmittel:12,5Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff imt den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Trcpfnässe. Nach dem Abtrocknen des Spritzbelages werden die Pflanzen mit einer wäßrigen Sporensuspension von Pyricularia oryzaè inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß der Herstellungsbeispiele: 1, 9, 12 und 11.

### Beispiel D

Pyricularia-Test (Reis)/ systemisch
Lösungsmittel: 12,5 Gewichtsteile Aceton
Emulgator: 0,3Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß der Herstellungsbeispiele: 1, 9, 14 und 12.

### Beispiel E

Pellicularia-Test (Reis)
Lösungsmittel: 12,5Gewichtsteile Aceton
Emulgator: 0,3 Gewichtsteile Alkylarylopolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäß der Herstellungsbeispiele: 1, 9, 14, 12 und 11.

### Beispiel F

Erysiphe-Test (Gerste) / protektiv
Lösungsmittel:¹⁰⁰Gewichtsteile Dimethylformamid
Emulgator: 0,25_{Gewichtsteile} Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele: 1, 5, 10, 11 und 12.

### Beispiel G

Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang_{'} mit dem Beizmittel in einer verschlossenen Glasflasche.

Die Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt in diesem Test z. B. die Verbindung gemäß Herstellungsbeispiel: 1.

### Beispiel _{H}

Drechslera graminea-Test (Gerste) / Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen in diesem Test z.B. die Verbindungen gemäß der Herstellungsbeispiele: 14 und 10.

## Patentansprüche

1. Azolyl-tetrahydrofuran-2-yliden-methan-Derivate der allgemeinen Formel (I) 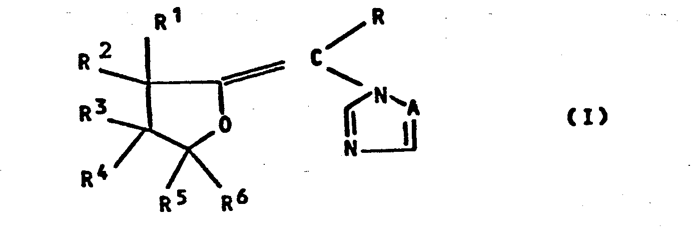 in welcher
A für ein Stickstoffatom oder die CH-Gruppe steht,
R für Alkyl, Alkenyl, Alkinyl, Alkoxyalkyl, Alkylthioalkyl, Halogenalkyl, Halogenalkoxyalkyl, Halogenalkylthioalkyl, Halogenalkenyl, gegebenenfalls substituiertes Aralkyl, gegebenenfalls substituiertes Aryloxyalkyl, gegebenenfalls substituiertes Arylthioalkyl, gegebenenfalls substituiertes Cycloalkyl oder gegebenenfalls substituiertes Cycloalkylalkyl steht,
R¹ bis _{R}⁶ gleich oder verschieden sind und für Wasserstoff, Alkyl, Halogenalkyl oder Halogen steht, wobei maximal bis zu 3 Substituenten für Halogen bzw. Halogenalkyl stehen, oder
Rund _{R} ² gemeinsam mit dem Kohlenstoffatom, an das sie gebunden sind für gegebenenfalls substituiertes Cycloalkyl stehen oder
R² und R³ gemeinsam mit den Kohlenstoffatomen, an die sie gebunden sind, für gegebenenfalls substituiertes Cycloalkyl stehen,
und deren Säureadditions-Salze und Metallsalz-komplexe.

2. Azolyl-tetrahydrofuran-2-yliden-methan-Derivate gemäß Anspruch 1, wobei in der Formel (I)
A für Stickstoffatom oder die CH-Gruppe steht;
R für geradkettiges oder verzweigtes Alkyl mit 1 bis 12 Kohlenstoffatomen, für geradkettiges oder verzweigtes Alkenyl und Alkinyl mit jeweils 2 bis 12 Kohlenstoffatomen, für geradkettiges oder verzweigtes Alkoxyalkyl und Alkylthioalkyl mit jeweils 1 bis 6 Kohlenstoffatomen in jedem Alkylteil, für Halogenalkyl, Halogenalkoxyalkyl und Halogenalkyl- thioalkyl mit jeweils 1 bis 6 Kohlenstoffatomen in jedem Alkylteil und mit jeweils 1 bis 6 gleichen oder verschiedenen Halogenatomen,für Halogenalkenyl mit 2 bis 6 Kohlenstoffatomen und 1 bis 5 gleichen oder verschiedenen Halogenatomen, ferner für jeweils gegebenenfalls einfach oder mehrfach, gleich oder verschieden im Phenylteil substituiertes Phenylalkyl, Phenoxyalkyl oder Phenylthioalkyl mit jeweils 1 bis 4 Kohlenstoffatomen im Alkylteil steht, wobei als Substituenten genannt seien: Halogen, Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 5 gleichen oder verschiedenen Halogenatomen; Nitro; Cyano; sowie gegebenenfalls durch Halogen substituiertes Phenyl und Phenoxy; sowie für jeweils gegebenenfalls einfach oder mehrfach durch Alkyl mit 1 bis 4 Kohlenstoffatomen substituiertes Cycloalkyl und Cycloalkylalkyl mit jeweils 3 bis 7 Kohlenstoffatomen im Cycloalkylteil und 1 bis 4 Kohlenstoffatomen im Alkylteil;
R bis _{R}⁶, die gleich oder verschieden sein können, für Wasserstoff, geradkettiges oder verzweigtes Alkyl mit 1 bis 4 Kohlenstoffatomen oder für Halogenalkyl mit 1 oder 2 Kohlenstoffatomen und 1 bis 5 gleichen oder verschiedenen Halogenatomen stehen, wobei maximal bis zu 3 Substituenten für Halogenalkyl stehen,
_{R}³ bis _{R}⁶, die gleich oder verschieden sein können, außerdem für Halogen stehen, wobei maximal bis zu 3 Substituenten diese Bedeutung haben; oder
R und R² gemeinsam mit dem Kohlenstoffatom, an das sie gebunden sind, für gegebenenfalls durch Alkyl mit 1 oder 2 Kohlenstoffatomen substituiertes Cycloalkyl mit 4 bis 7 Kohlenstoffatomen stehen oder
R² und R³ gemeinsam mit den Kohlenstoffatomen, an die sie gebunden sind, für gegebenenfalls durch Alkyl mit 1 oder 2 Kohlenstoffatomen substituiertes Cycloalkyl mit 4 bis 7 Kohlenstoffatomen stehen
und deren Säureadditions-Salze und Metallsalz-komplexe.

3. Azolyl-tetrahydrofuran-2-yliden-methan-Derivate gemäß Anspruch 1, wobei in der Formel (I)
A für ein Stickstoffatom oder die CH-Gruppe steht;
R für geradkettiges oder verzweigtes Alkyl mit 1 bis 8 Kohlenstoffatomen, für geradkettiges oder verzweigtes Alkenyl und Alkinyl mit jeweils 2 bis 6 Kohlenstoffatomen, für geradkettiges oder verzweigtes _{Al}- koxyalkyl und Alkylthioalkyl mit jeweils 1 bis 4 Kohlenstoffatomen in jedem Alkylteil, für Halogenalkyl, Halogenalkoxyalkyl und Halogenalkyl- > thioalkyl mit jeweils 1 bis 4 Kohlenstoffatomen in jedem Alkylteil und mit jeweils 1 bis 5 Fluor-und/oder Chloratomen, für Halogenalkenyl mit 2 bis 4 Kohlenstoffatomen und 1 bis 3 Fluor- und/ oder Chloratomen; ferner für jeweils gegebenenfalls einfach bis dreifach, gleich oder verschieden im Phenylteil substituiertes Phenylalkyl, Phenoxyalkyl oder Phenylthioalkyl mit jeweils 1 oder 2 Kohlenstoffatomen im Alkylteil steht, wobei als Substituenten genannt seien: Fluor, Chlor, Methyl, Ethyl, Isopropyl, tert.-Butyl, Methoxy, Methylthio, Trifluormethyl, Trifluormethoxy, Trifluormethylthio, Nitro, Cyano, sowie gegebenenfalls durch Chlor substituiertes Phenyl und Phenoxy; sowie auch für jeweils gegebenenfalls durch Methyl und/oder Ethyl substituiertes Cyclohexyl oder Cyclohexylmethyl steht;
R bis R⁶, die gleich oder verschieden sein können, für Wasserstoff, Methyl, Ethyl oder Halogenmethyl mit 1 bis 3 gleichen oder verschiedenen Halogenatomen stehen, wobei maximal drei Substituenten für Halogenmethyl stehen; oder
R bis _{R}⁶, die gleich oder verschieden sein können, außerdem für Fluor, Chlor oder Brom stehen, wobei maximal bis zu drei Substituenten diese Bedeutung haben; oder
R¹ und R² gemeinsam mit dem Kohlenstoffatom, an das sie gebunden sind, für Cyclopentyl oder Cyclohexyl stehen; und
R² und R³ gemeinsam mit den Kohlenstoffatomen, an die sie gebunden sind, für Cyclopentyl oder Cyclohexyl stehen und deren Säureadditions-Salze und Metallsalz-Komplexe.

Verfahren zur Herstellung von Azolyl-tetrahydrofuran-2-ylidenmethan-Derivaten der allgemeinen Formel (I) 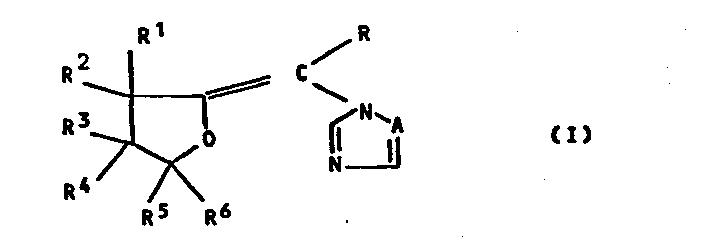 in welcher
A für ein Stickstoffatom oder die CH-Gruppe steht,
R für Alkyl, Alkenyl, Alkinyl, Alkoxyalkyl, Alkylthioalkyl, Halogenalkyl, Halogenalkoxyalkyl, Halogenalkylthioalkyl, Halogenalkenyl, gegebenenfalls substituiertes Aralkyl, gegebenenfalls substituiertes Aryloxyalkyl, gegebenenfalls substituiertes Arylthioalkyl,^{:}gegebenenfalls substituiertes Cycloalkyl oder gegebenenfalls substituiertes Cycloalkylalkyl steht,
R¹ bis _{R}⁶ gleich oder verschieden sind und für Wasserstoff, Alkyl, Halogenalkyl oder Halogen stehen, wobei maximal bis zu 3 Substituenten für Halogen bzw. Halogenalkyl stehen, oder
R und R² gemeinsam mit dem Kohlenstoffatom, an das sie gebunden sind,für gegebenenfalls substituiertes Cycloalkyl stehen oder
_{R}² und R³ gemeinsam mit den Kohlenstoffatomen, an die sie gebunden sind, für gegebenenfalls substituiertes Cycloalkyl stehen,
und deren Säureadditions-Salze und Metallsalz-Komplexe, dadurch gekennzeichnet, daß man Azolyl-(2-hydroxytetrahydrofuran-2-yl)-methan-Derivate der Formel (II) 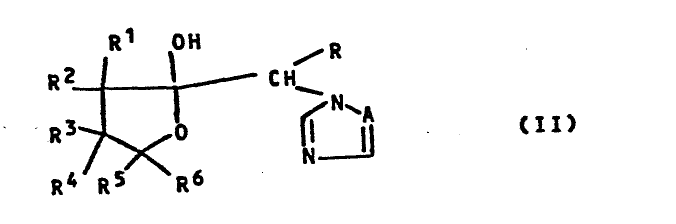 in welcher
A, R und R¹ bis R⁶ die oben angegebene Bedeutung haben,
in Gegenwart eines Verdünnungsmittels und in Gegenwart eines sauren Katalysators zur Wasserabscheidung erhitzt und an die so erhaltenen Verbindungen der Formel (I) gegebenenfalls anschließend eine Säure oder ein Metallsalz addiert.

5. Azolyl-(2-hydroxytetrahydrofuran-2-yl)-methan-Derivate der allgemeinen Formel (II) 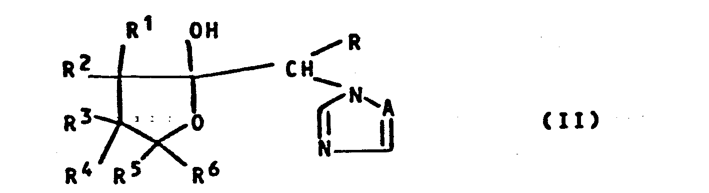 in welcher
A für ein Stickstoffatom oder die CH-Gruppe steht,
R für Alkyl, Alkenyl, Alkinyl, Alkoxyalkyl, Alkylthioalkyl, Halogenalkyl, Halogenalkoxyalkyl, Halogenalkylthioalkyl, Halogenalkenyl, gegebenenfalls substituiertes Aralkyl, gegebenenfalls substituiertes Aryloxyalkyl, gegebenenfalls substituiertes Arylthioalkyl, gegebenenfalls substituiertes Cycloalkyl oder gegebenenfalls substituiertes Cycloalkylalkyl steht,
R bis R gleich oder verschieden sind und für Wasserstoff, Alkyl, Halogenalkyl oder Halogen stehen, wobei maximal bis zu 3 Substituenten für Halogen bzw. Halogenalkyl stehen, oder
R¹ und R² gemeinsam mit dem Kohlenstoffatom, an das sie gebunden sind,für gegebenenfalls substituiertes Cycloalkyl stehen oder
R² und R³ gemeinsam mit den _{K}öhlenstoffatomen, an die sie gebunden sind, für gegebenenfalls substituiertes Cycloalkyl stehen.

6. 2-Azolylmethyl-2-hydroxy-tetrahydrofurane der Formel (III) 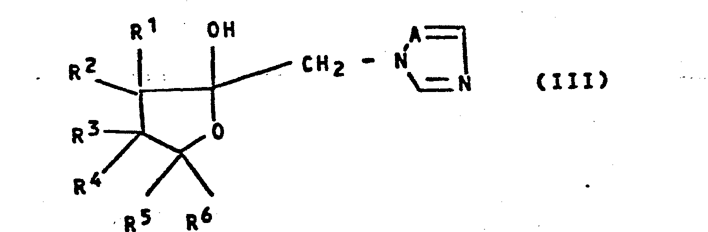 in welcher
A für ein Stickstoffatom oder die CH-Gruppe steht,
R¹ bis R⁶ gleich oder verschieden sind und für Wasserstoff, Alkyl, Halogenalkyl oder Halogen stehen, wobei maximal bis zu 3 Substituenten für Halogen bzw. Halogenalkyl stehen, oder
R¹ und _{R}² gemeinsam mit dem Kohlenstoffatom, an das sie gebunden sind für gegebenenfalls substituiertes Cycloalkyl stehen oder
_{R}² und _{R}³ gemeinsam mit den Kohlenstoffatomen, an die sie gebunden sind, für gegebenenfalls substituiertes Cycloalkyl stehen.

Substituierte 2-Methylen-tetrahydrofurane der Formel 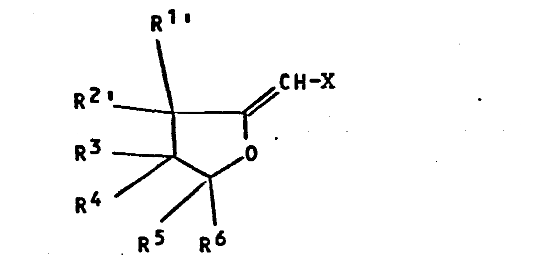 in welcher
X für Chlor, 1,2,4-Triazolyl oder Imidazolyl steht,
R1' und R²' gleich oder verschieden sind und für Wasserstoff, Alkyl, Halogenalkyl oder Halogen stehen,
R³ bis R⁶ gleich oder verschieden sind und für Wasserstoff, Alkyl, Halogenalkyl oder Halogen stehen, wobei R^{1'} und R^{2'} nicht gleichzeitig für Methyl stehen, wenn R³ bis R⁶ gleichzeitig für Wasserstoff stehen.

8. Halogenketone der Formel (VIa) 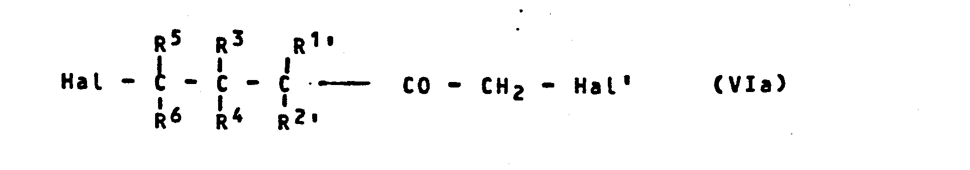 in welcher
Hal und Hall für Halogen,
- R^{1'}und R^{2'} gleich oder verschieden sind und für Wasserstoff, Alkyl, Halogenalkyl oder Halogen stehen,
_{R}³ bis R⁶ gleich oder verschieden sind und für Wasserstoff, Alkyl, Halogenalkyl oder Halogen stehen, wobei R^{1'} und R²⁸ nicht gleichzeitig für Methyl stehen, wenn R³ bei R⁶ gleichzeitig für Wasserstoff stehen.

9. Fungizide Mittel, gekennzeichnet durch einen Gehalt an mindestens einem Azolyl-tetrahydrofuran-2-yliden- methan-Derivat der Formel (I) in Ansprüchen 1 und 4.

10. Verfahren zur Bekämpfung von Pilzen, dadurch gekennzeichnet, daß man Azolyl-tetrahydrofuran-2-yliden- methan-Derivate der Formel (I) in Ansprüchen 1 und 4 auf Pilze oder ihren Lebensraum einwirken läßt.

11. Verwendung von Azolyl-tetrahydrofuran-2-yliden- methän-Derivaten der Formel (I) in Ansprüchen 1 und 4 als Pflanzenschutzmittel.

12. Verwendung von Azolyl-tetrahydrofuran-2-yliden- methan-Derivaten der Formel (I) in Ansprüchen 1 und 4 zur Bekämpfung von Pilzen.

13. Verfahren zur Herstellung von fungiziden Mitteln, dadurch gekennzeichnet, daß man Azolyl-tetrahydrofuran-2-yliden-methan-Derivate der Formel (I) in Ansprüchen 1 und 4 mit Streckmitteln und/oder oberflächenaktiven Mitteln vermischt.
